# EUROPÄISCHE PATENTSCHRIFT

(11) **EP 1 362 579 B1**
(45) Veröffentlichungstag und Bekanntmachung des Hinweises auf die Patenterteilung: **24.05.2006**
(21) Anmeldenummer: 03008968.4
(22) Anmeldetag: 17.04.2003
(51) Int. Cl.: A61K 8/49, A61Q 5/10

(54) **Mittel zum Färben von keratinhaltigen Fasern**
Agent for dyeing keratinous fibres
Agent pour teindre des fibres kératiniques

(30) Priorität: 26.04.2002 DE 10218690
(43) Veröffentlichungstag der Anmeldung: 19.11.2003
(73) Patentinhaber: Henkel Kommanditgesellschaft auf Aktien, 40589 Düsseldorf (DE)
(72) Erfinder: Möller, Hinrich, Dr., 40789 Monheim (DE); Oberkobusch, Doris, Dr., 40591 Düsseldorf (DE); Höffkes, Horst, Dr., 40595 Düsseldorf (DE)

(56) Entgegenhaltungen:
- WO-A-93/19725
- DE-A- 4 314 318
- DE-A- 19 717 282
- US-A- 5 190 564

## Beschreibung

Die Erfindung betrifft ein Mittel zum Färben von keratinhaltigen Fasern, insbesondere menschlichen Haaren, das als Isatinderivat mindestens eine Bisisatinverbindung und/oder ein Isatin mit einer, gegebenenfalls über ein aromatisches System, ankondensierten 1H-Pyrrol-2,3-dion-Struktureinheit enthält, die Verwendung dieser Verbindungen in Mitteln zum Färben von keratinhaltigen Fasern sowie ein Verfahren zum Färben von keratinhaltigen Fasern, insbesondere menschlichen Haaren.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarostoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Aus der Druckschrift DE-A1-197 17 282 sind Isatinderivat-haltige Haarfärbemittel bekannt. Die in diesen Färbemitteln verwendeten Isatinderivate tragen am Stickstoffatom des Isatingrundgerüstes eine -CH₂-Y Gruppe, worin Y eine Hydroxygruppe, eine C₁-C₄₋Alkoxygruppe oder eine Aminogruppe bedeutet. Die letztgenannte Aminogruppe kann unter anderem Bestandteil eines heterocyclischen Ringes sein.

Färbemittel, die als Isatinderivat mindestens eine Bisisatinverbindung und/oder eine Isatinverbindung mit einer, gegebenenfalls über ein aromatisches System, ankondensierten 1H-Pyrrol-2,3-dion-Struktureinheit enthalten, sowie deren Verwendung zum Färben von keratinhaltigen Fasern sind bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel I dargestellten IsatinDerivate sich auch in Abwesenheit von oxidierenden Agenzien hervorragend zum Färben von keratinhaltigen Fasern eignen. Sie ergeben Ausfärbungen mit hervorragender Brillanz und Farbtiefe und führen zu vielfältigen Farbnuancen. Es werden insbesondere Ausfärbungen über einen Nuancenbereich von gelb über gelbbraun, orange, braunorange, braun, rot, rotviolett bis hin zu blauviolett und schwarz erhalten. Der Einsatz von oxidierenden Agentien soll jedoch nicht prinzipiell ausgeschlossen werden.

Gegenstand der Erfindung ist ein Mittel zum Färben von keratinhaltigen Fasern, insbesondere menschlichen Haaren, enthaltend mindestens eine Verbindung gemäß Formel I und/oder deren physiologisch verträglichen Salze 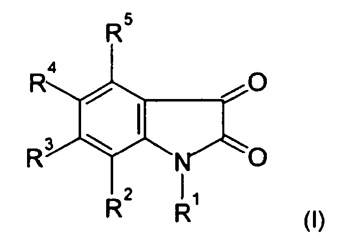 wobei
- R¹ steht für ein Wasserstoffatom, eine C₁-C₄-Alkylgruppe, C₂-C₄-Alkenylgruppe, eine Arylgruppe, eine Aryl-C₁-C₄-alkylgruppe, eine C₁-C₄-Acylgruppe, eine C₁-C₄₋Hydroxyalkylgruppe, eine C₂-C₄-Dihydroxyalkylgruppe, eine C₁-C₄-Sulfoalkylgruppe, eine C₁-C₄-Carboxyalkylgruppe, eine Gruppe R^{I}R^{II}N-(CH₂)ₘ- oder R^{I}R^{II}R^{III}N⁺-(CH₂)ₘ-, worin m ist gleich 1, 2, 3 oder 4 und R^{I}, R^{II} und R^{III} stehen unabhängig voneinander für ein Wasserstoffatom, eine C₁-C₄-Alkylgruppe, eine C₁-C₄-Hydroxyalkylgruppe, eine Aryl-C₁-C₄-alkylgruppe oder R^{I} und R^{II} bilden gemeinsam mit dem Stickstoffatom einen heterozyklischen 5-, 6- oder 7-Ring, oder eine Gruppe gemäß Formel II, 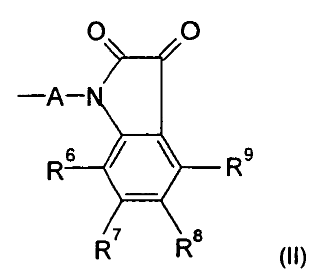 wobei
   - A steht für eine C₁-C₈-Alkylengruppe, eine Carbonylgruppe, eine Arylengruppe oder eine Gruppe -(CH₂)ⱼ-O-(CH₂)ₖ-, in der j und k stehen unabhängig voneinenader für eine Zahl 1, 2, 3 oder 4,
   - R⁶, R⁷, R⁸ und R⁹ stehen unabhängig voneinander für ein Wasserstoffatom, ein Halogenatom, eine Nitrogruppe, eine Sulfonsäuregruppe, eine Hydroxygruppe, eine Carboxylgruppe, eine C₁-C₄-Alkylgruppe, eine Arylgruppe, eine C₁-C₄₋Alkoxygruppe, eine C₁-C₄-Hydroxyalkylgruppe, eine C₂-C₄₋Dihydroxyalkylgruppe, eine C₁-C₄-Sulfoalkylgruppe, eine C₁-C₄₋Carboxyalkylgruppe, eine C₁-C₄-Acylgruppe, eine Gruppe R^{IV}R^{V}N-(CH₂)ₙ- oder eine Gruppe R^{IV}R^{V}R^{VI}N⁺-(CH₂)ₙ-, worin R^{IV}, R^{V} und R^{VI} stehen unabhängig voneinander für ein Wasserstoffatom, eine C₁-C₄-Alkylgruppe, eine C₁-C₄₋Hydroxyalkylgruppe oder eine Aryl-C₁-C₄-alkylgruppe und n ist gleich 1, 2, 3 oder 4, und
- R², R³, R⁴ und R⁵ stehen unabhängig voneinander für ein Wasserstoffatom, ein Halogenatom, eine C₁-C₄-Alkylgruppe, eine Arylgruppe, eine C₁-C₄-Alkoxygruppe, eine C₁-C₄-Hydroxyalkylgruppe, eine C₂-C₄-Dihydroxyalkylgruppe, eine C₁-C₄₋Sulfoalkylgruppe, eine C₁-C₄-Carboxyalkylgruppe, eine C₁-C₄-Acylgruppe, eine Gruppe R^{VII}R^{VIII}N-(CH₂)_{q}- sowie eine Gruppe R^{VII}R^{VIII}R^{IX}N⁺-(CH₂)_{q}-, worin R^{VII}, R^{VIII} und R^{IX} stehen unabhängig voneinander für ein Wasserstoffatom, eine C₁-C₄₋Alkylgruppe, eine C₁-C₄-Hydroxyalkylgruppe oder eine Aryl-C₁-C₄-alkylgruppe und q ist gleich 1, 2, 3 oder 4, eine Nitrogruppe, eine Sulfonsäuregruppe, eine Hydroxygruppe, eine Carboxylgruppe, eine Gruppe gemäß Formel II, in der A, R⁶, R⁷, R⁸ und R⁹ wie oben definiert sind oder eine Gruppe gemäß Formel III,
   wobei zwei benachbarte Reste aus R², R³, R⁴ und R⁵ einen ankondensierten Rest bilden können, der ausgewählt ist aus den Formeln IV bis VIII, 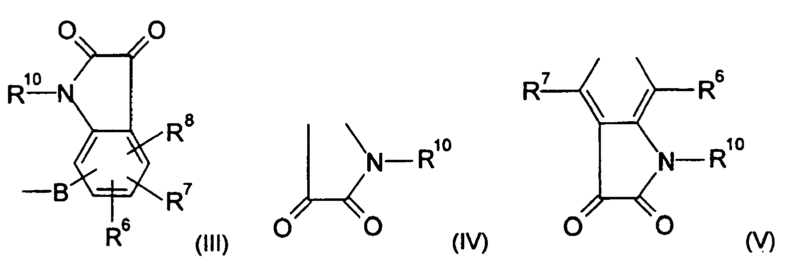 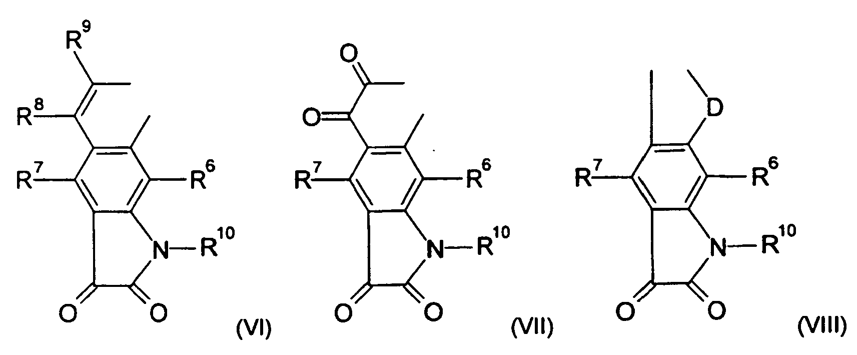 wobei
   - R⁶, R⁷, R⁸ und R⁹ wie oben definiert sind,
   - R¹⁰ steht für ein Wasserstoffatom, eine C₁-C₄-Alkylgruppe, C₂-C₄₋Alkenylgruppe, eine Arylgruppe, eine Aryl-C₁-C₄-alkylgruppe, eine C₁-C₄₋Acylgruppe, eine C₁-C₄-Hydroxyalkylgruppe, eine C₂-C₄-Dihydroxyalkylgruppe, eine C₁-C₄-Sulfoalkylgruppe, eine C₁-C₄-Carboxyalkylgruppe, eine Gruppe R^{X}R^{XI}N-(CH₂)ₛ- oder R^{X}R^{XI}R^{XII}N⁺-(CH₂)ₛ-, worin R^{X}, R^{XI} und R^{XII} stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe und s ist gleich 1, 2, 3 oder 4,
   - B steht für eine direkte Bindung, ein Sauerstoffatom, ein Schwefelatom, eine Disulfidgruppe, eine Gruppe NH, eine Sulfonylgruppe, eine C₁-C₈₋Alkylengruppe, eine Carbonylgruppe, eine Gruppe -O-(CH₂)ₜ-O-, in der t gleich 1, 2, 3 oder 4 ist, eine Gruppe -(CH₂)ᵤ-O-(CH₂)ᵥ-, in der u und v unabhängig voneinander einen Wert von 1, 2, 3 oder 4 haben,
   - D steht für eine Methylengruppe, eine Carbonylgruppe und eine Gruppe NH mit der Maßgabe, daß entweder einer der Reste R¹, R², R³, R⁴ oder R⁵ nach der obigen Definition eine Gruppe der Formeln II oder III ist, oder zwei benachbarte Reste aus R², R³, R⁴ oder R⁵ gemeinsam einen ankondensierten Rest bilden, der ausgewählt ist aus den Formeln IV bis VIII.

Die vorgenannten Verbindungen gemäß Formel I können auch in Form ihrer Hydrate Anwendung finden. Sollten die Verbindungen als physiologisch verträgliche Salze zum Einsatz kommen, werden bevorzugt Kationen der Alkali-, oder Erdalkalimetalle sowie Ammoniumionen verwendet, wenn die Verbindung der Formel I, insbesondere durch Carboxy- oder Sulfogruppen tragende Substituenten, als Anion vorliegt. Die physiologisch verträglichen Salze der Verbindungen der Formel I können gemäß Formel I durch quaternierte Aminosubstituenten gebildet werden. In diesem Falle werden die Gegenanionen bevorzugt aus der Gruppe ausgewählt, die gebildet wird aus Benzdsulfonat, p-Toluolsulfonat, Methansulfonat, Perchlorat, Sulfat, Chlorid, Bromid, lodid und Tetrachlorzinkat,.

Beispiele für die als Substituenten im Rahmen dieser Anmeldung genannten C₁-C₄₋Alkylreste sind die Gruppen Methyl, Ethyl, Propyl, Isopropyl, n-Butyl, sec-Butyl und tert.-Butyl. Ethyl und Methyl sind bevorzugte Alkylreste. Als C₂-C₄-Alkenylgruppen finden bevorzugt die Vinyl- und die Allylgruppe Verwendung. Erfindungsgemäß bevorzugte C₁₋C₄-Alkoxyreste sind beispielsweise eine Methoxy- oder eine Ethoxygruppe. Weiterhin können als bevorzugte Beispiele für eine C₁-C₄-Hydroxyalkylgruppe eine Hydroxymethyl, eine 2-Hydroxyethyl-, eine 2-Hydroxypropyl, eine 3-Hydroxypropyl- und eine 4-Hydroxybutylgruppe genannt werden; wobei die 2-Hydroxyethylgruppe besonders bevorzugt ist. Die Methylen-, Ethylen-, Propylen-, Butylen-, Pentylen- und Hexylengruppe sind bevorzuge C₁-C₈-Alkylengruppen. Als bevorzugte C₂-C₄₋Dihydroxyalkylgruppen gelten die 1,2-Dihydroxyethylgruppe und die 2,3-Dihydroxypropylgruppe, wobei die 2,3-Dihydroxypropylgruppe besonders bevorzugt ist. Bevorzugte Arylgruppen sind Phenyl, Naphthyl und Biphenyl. Beispiele für Arylengruppen sind Phenylen und Naphthylen. Beispiele für bevorzugte Halogenatome sind F-, Cl-, oder Br-Atome, wobei Cl-Atome ganz besonders bevorzugt sind. Als bevorzugte Beispiele für eine Aminoalkylen-Gruppe, die allgemein in den Formeln I bis VIII als eine RR'N-(CH₂)ₓ-Gruppe bezeichnet wird, gelten die Aminomethyl-, die Aminoethyl, die Aminopropyl-, die N,N-Dimethylaminomethyl-, die N,N-Dimethylaminoethyl-, die N,N-Dimethylaminomethyl-, die 2-(N,N-Diethylamino)-ethyl-, die N,N-Bis(2-hydroxyethyl)aminomethyl- und die 2-[N,N-Bis(2-hydroxyethyl)amino]-ethylgruppe. Bevorzugte C₁-C₄-Acylgruppen sind Formyl, Acetyl, Propionyl und Butyryl. Eine bevorzugte C₁-C₄-Carboxyalkylgruppe ist die 3-Carboxypropylgruppe. Bevorzugte Sulfo-C₁-C₄-alkylgruppen sind die 2-Sulfoethyl-, die 2-Sulfopropyl- und die 3-Sulfopropylgruppe. Beispiele für eine Aryl-C₁-C₄-alkylgruppe sind Benzyl und 2-Phenylethyl. Unter funktionellen Carbonylderivaten werden Kondensationsprodukte aus Carbonylverbindungen und Verbindungen mit einer NH₂-Funktionalität verstanden. Beispiele für funktionelle Carbonylderivate sind Oxime und Imine. Die weiteren verwendeten Begriffe leiten sich erfindungsgemäß von den hier gegebenen Definitionen ab.

Bevorzugte Isatinderivate gemäß Formel I sind ausgewählt aus (1H, 7H)-2,3,5,6-Tetrahydro-pyrrolo[3,2-f]indol-2,3,5,6-tetron, (1H, 6H)-2,3,7,8-Tetrahydropyrrolo[2',3':1,2]-naphtho[5,6-b]pyrrol-2,3,7,8-tetron, (3H, 8H)-1,2,6,7-Tetrahydro-5,10-dichlor-indolo[7,6-g]indol-1,2,6,7-tetron, 5,5'-Oxy-bis(1H-2,3-dihydro-indol-2,3-dion), 6,6'-Oxy-bis(1H-2,3-dihydro-indol-2,3-dion), 6,6'-Carbonyl-bis-(1H-2,3-dihydro-indol-2,3-dion), (1H, 7H)-2,3,5,6-Tetrahydro-8-chlor-4-methoxy-benzo[1,2-b:5,4-b']dipyrrol-2,3,5,6-tetron, (1H, 8H)-2,3,6,7-Tetrahydro-benzo[2,1-b:3,4-b']dipyrrol-2,3,6,7-tetron, 6,6'-Methylen-bis(1 H-2,3-tetrahydro-indol-2,3-dion), 6,6'-Methylen-bis(H-2,3-tetrahydo-7-chlor-indol-2,3-dion), 1,1'-Methylen-bis(1 H-2,3-tetrahydo-indol-2,3-dion), 1,1'-(1,3-Propandiyl)-bis(1H-2,3-tetrahydro-5-methyl-indol-2,3-dion), (1H, 7H)-2,3,5,6-tetrahydro-8-methyl-benzo[1,2-b:5,4-b']dipyrrol-2,3,5,6-tetron, (1H, 1'H)-2,2',3,3'-tetrahydro-7,7'-dimethoxy-5,5'-biindol-2,2',3,3'-tetron, (1H, 7H)-2,3,5,6-Tetrahydro-4,8-dimethylbenzo[1,2-b:5,4-b']dipyrrol-2,3,5,6-tetron, (1H, 1'H)-2,2',3,3'-Tetrahydro-5,5',6,6' tetrahydroxy-1,1'-dimethyl-4,4'-biindol-2,2',3,3'-tetron.

Die Verbindungen mit der Formel I sind literaturbekannt und nach bekannten Syntheseverfahren herstellbar.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Die voranstehend genannten Verbindungen mit der Formel I werden vorzugsweise in den erfindungsgemäßen Mitteln jeweils in einer Menge von 0,03 bis 65 mmol, insbesondere von 1 bis 40 mmol, bezogen auf 100 g des gesamten Färbemittels, verwendet.

Färbungen mit noch erhöhter Brillanz und verbesserten Echtheitseigenschaften (Lichtechtheit, Waschechtheit, Reibechtheit) über einen weiten Nuancenbereich werden erzielt, wenn die erfindungsgemäß eingesetzten Verbindungen der Formel I gemeinsam mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aromatischen Aminen, stickstoffhaltigen heterocyclischen Verbindungen und aromatischen Hydroxyverbindungen, Aminosäuren und deren physiologisch verträglichen Salzen, sowie anderen CH-aciden Verbindungen verwendet werden. Dies sind einerseits Verbindungen, die für sich alleine keratinhaltige Fasern nur schwach färben und erst gemeinsam mit den Verbindungen der Formeln I brillante Färbungen ergeben. Andererseits sind darunter aber auch Verbindungen, die bereits als Oxidationsfarbstoffvorprodukte eingesetzt werden.

Geeignete Verbindungen mit primärer oder sekundärer Aminogruppe als Komponente B sind z. B. primäre und sekundäre aromatische Amine wie N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)-2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Dian:ino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxybenzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthafin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 2,2'-(1,4-Piperazinylen-1,4-dimethylen)-bis-4-aminophenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxynaphth-2-yl]-trimethylammoniumchlorid, [8-((4-Amino-3-nitrophenyl)-azo)-7-hydroxynaphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel IX dargestellt sind 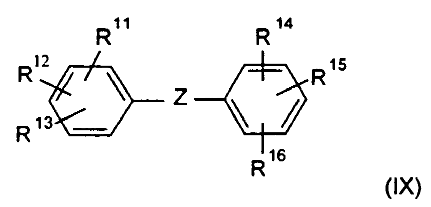 in der
- R¹¹ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
- R¹², R¹³, R¹⁴, R¹⁵ und R¹⁶ unabhängig voneinander für ein Wasserstoffatom, eine Hydroxy- oder eine Aminogruppe, die durch C₁-C₄-Alkyl-, C₁-C₄-Hydroxyalkyl, C₁-C₄₋Alkoxy-, C₁-C₄-Aminoalkyl- oder C₁-C₄-Alkoxy-C₁-C₄-alkylgruppen substituiert sein kann, stehen, und
- Z für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel X 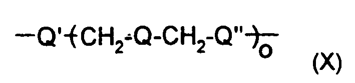 in der
   - Q eine direkte Bindung, eine CH₂- oder CHOH-Gruppe bedeutet,
   - Q' und Q" unabhängig voneinander für ein Sauerstoffatom, eine NR¹⁷-Gruppe, worin R¹⁷ ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ-O, worin p und p' 2 oder 3 sind, stehen und
   - o eine Zahl von 1 bis 4 bedeutet,

wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenylamino)-ethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Als Aminosäuren kommen bevorzugt alle natürlich vorkommenden und synthetischen α-Aminosäuren in Frage, z.B. die durch Hydrolyse aus pflanzlichen oder tierischen Proteinen, z.B. Kollagen, Keratin, Casein, Elastin, Sojaprotein, Weizengluten oder Mandelprztein zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Prolin, Lysin und Tryptophan. Aber auch andere Aminosäuren, wie z.B. 6-Aminocapronsäure und β-Alanin, können eingesetzt werden.

Als CH-acide Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Methyl-2-chinaldinium-p-toluolsulfonat, 1 -Ethyl-2-chinaldiniumiodid, 1-Ethyl-2-chinaldinium-p-toluolsulfonat, 1,4-Dimethylchinoliniumiodid, 1,4-Dimethylchinolinium-p-toluolsulfonat, Barbitursäure, Thiobarbitursäure, 1,3-Dimethylthiobarbitursäure, 1,3-Diethylthiobarbitursäure, Oxindol, 3-Indoxylacetat, 2-Cumaranon, 5-Hydroxy-cumaranon, 6-Hydroxy-cumaranon, 1-Methyl-3-phenyl-2-pyrazolinon, Indan-1,3-dion, Indan-1-on, Benzoylacetonitril, 1-Dicyanmethylenindan, 1,3-Bis(dicyanmethylen)indan, 3-Dicyanmethylenindan-1-on, 1,3-Diiminoisoindolin, 2-Amino-4-imino-1,3-thiazolin-hydrochlorid, 3-Cyan-1,4-dimethyl-6-hydroxy-2-pyridon und 3-Cyan-1-ethyl-6-hydroxy-4-methyl-2-pyridon.

Die Verbindungen der Komponente B werden besonders bevorzugt ausgewählt aus der Gruppe bestehend aus N-(2-Hydroxyethyl)-N-ethyl-p-phenylendiamin, 2-Chlor-p-phenylendiamin, N,N-Bis-(2-hydroxyethyl)-p-phenylendiamin, 2-Aminophenol, 3-Aminophenol, 4-Aminophenol, 2-Amino-6-chlor-4-nitrophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlorphenol, 2-Methyl-5-aminophenol, 3-Methyl-4-aminophenol, 2-Methyl-5-(2-hydroxyethylamino)phenol, 2-Methyl-5-amino-4-chlorphenol, 6-Methyl-3-amino-2-chlorphenol, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 2,6-Bis(2-hydroxyethylamino)-1-methylbenzol, Bis-(2-hydroxy-5-aminophenyl)methan, Bis-(4,5-amino-2-hydroxyphenyl)-methan, 1,3-Bis(2,4-diaminophenoxy)propan, 1,4-Bis(4-aminophenyl)-1,4-diazacycloheptan, 1,8-Bis(2,5-Diaminophenoxy)-3,6-dioxaoctan, 4,4'-Diaminodiphenylamin, 3,4-Methylendioxyphenol, 3,4-Diaminobenzoesäure, 2,5-Diaminopyridin, 2-Dimethylamino-5-aminopyridin, 2-Amino-3-hydroxypyridin, 3-Amino-2-methylamino-6-methoxypyridin, 2,3-Diamino-6-methoxypyridin, 3,5-Diamino-2,6-dimethoxypyridin, 2,6-Dihydroxy-3,4-dimethylpyridin, 2-Hydroxy-4,5,6-triaminopyrimidin, 4-Hydroxy-2,5,6-triaminopyrimidin, 2,4,5,6-Tetraaminopyrimidin, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol, 5,6-Dihydroxyindolin, 4-Amino-2,3-dimethyl-1-phenyl-3-pyrazolin-5-on (4-Aminoantipyrin), 2,3-Dimethylbenzothiazolium-p-toluolsulfonat, 1,2,3,3-Tetramethyl-3H-indolinium-p-toluolsulfonat, Thiobarbitursäure, Rhodanin, 2,3-Dimethyl-benzothiazolium-p-toluolsulfonat, 1,2-Dimethyl-naphtho[1,2-d]thiazolium-p-toluolsulfonat, 1-Methyl-2-chinaldinium-p-toluolsulfonat, 1,4-Dimethylchinolinium-p-toluolsulfonat, β-Alanin, L-Prolin, L-Lysin, DL-Tyrosin sowie deren vorzugsweise mit anorganischen Säuren gebildeten physiologisch verträglichen Salze.

Ganz besonders bevorzugte Verbindungen der Komponente B sind erfindungsgemäß N,N-Bis(2-hydroxyethyl)-p-phenylendiamin, 2,5-Diaminotoluol, 2-(2,5-Diaminophenyl)-ethanol, 2-Aminophenol, 3-Aminophenol, 4-Aminophenol, 2-Aminomethyl-4-aminophenol, 2-(Diethylaminomethyl)-4-aminophenol, 1-Hydroxy-2-amino-5-methylbenzol, 2,4-Diaminophenoxyethanol, 3-Amino-2,4-dichlorphenol, 2-Methyl-5-aminophenol, 2-Methyl-5-(2-hydroxyethylamino)-phenol, 6-Methyl-3-amino-2-chlorphenol, 2-Methyl-5-amino-4-chlorphenol, 3,4-Methylendioxyanilin, 3,4-Diaminobenzoesäure, 3,5-Diamino-2-methoxy-1-methylbenzol, 2-Amino-4-(2-hydroxyethylamino)anisol, 2,6-Bis-(2-hydroxyethylamino)-1-methylbenzol, 1,3-Bis-(2,4-diaminophenoxy)propan, 4,5-Diamino-1-(2-hydroxyethyl)pyrazol, 1,4-Bis(4-aminophenyl)-1,4-diazacycloheptan, Bis-(2-hydroxy-5-aminophenyl)methan, 1,8-Bis-(2,5-diaminophenoxy)-3,6-dioxaoctan, 4,4'-Diaminodiphenylamin, 2-Amino-3-hydroxypyridin, 3-Amino-2-methylamino-6-methoxypyridin, 2,6-Dihydroxy-3,4-dimethylpyridin, 5,6-Dihydroxyindol, 5,6-Dihydroxyindolin, 2,4,5,6-Tetraaminopyrimidin, 2-Hydroxy-4,5,6-triamino-pyrimidin, 4-Hydroxy-2,5,6-triamino-pyrimidin sowie deren mit vorzugsweise anorganischen Säuren gebildeten physiologisch verträglichen Salze.

Die voranstehend genannten Verbindungen der Komponente B können in einer Menge von jeweils 0,03 bis 65 mmol, insbesondere 1 bis 40 mmol, jeweils bezogen auf 100 g des gesamten Färbemittels, eingesetzt werden.

Unter die voranstehend beschriebene Ausführungsform fällt ebenfalls die Verwendung der Reaktionsprodukte der Isatinderivate mit der Formel I und der Komponente B als direktziehende Färbemittel, bevorzugt in einer Menge von 0,03 bis 65 mmol, insbesondere 1 bis 40 mmol, jeweils bezogen auf 100 g des gesamten Färbemittels. Derartige Reaktionsprodukte können z. B. durch kurzes Erwärmen der beiden Komponenten in stöchiometrischen Mengen in wässrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können zusätzlich in einer Menge von jeweils 0,03 bis 65 mmol, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann dabei verzichtet werden. Es kann jedoch unter Umständen wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkail- und Erdalkalimetalle oder aus lodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze oder Metalloxide, die einen leichten Wechsel zwischen zwei Oxidationsstufen der Metallionen ermöglichen. Beispiele sind Salze oder Oxide von Eisen, Ruthenium, Mangan und Kupfer.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red BN, Basic Red 76, HC Blue 2, Disperse Blue 3, Basic Blue 99, Disperse Violet 1, Disperse Violet 4, Disperse Black 9, Basic Brown 16 und Basic Brown 17 bekannten Verbindungen sowie 6-Nitro-1,2,3,4-tetrahydrochinoxalin, 4-N-Ethyl-1,4-bis-(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3C-Atomen in der Alkanolgruppe,
- lineare Fettsäuren mit 10 bis 22C-Atomen (Seifen),
- Ethercarbonsäuren der Formel R-O-(CH₂CH₂O)ₓ-CH₂-COOH, in der R eine lineare Alkylgruppe mit 10 bis 22C-Atomen und x = 0 oder 1 bis 16 ist,
- Acylsarcoside mit 10 bis 18C-Atomen in der Acylgruppe,
- Acyltauride mit 10 bis 18C-Atomen in der Acylgruppe,
- Acylisethionate mit 10 bis 18C-Atomen in der Acylgruppe,
- Sulfobernsteinsäuremono- und -dialkylester mit 8 bis 18C-Atomen in der Alkylgruppe und Sulfobernsteinsäuremono-alkylpolyoxyethylester mit 8 bis 18C-Atomen in der Alkylgruppe und 1 bis 6 Oxyethylgruppen,
- lineare Alkansulfonate mit 12 bis 18C-Atomen,
- lineare Alpha-Olefinsulfonate mit 12 bis 18C-Atomen,
- Alpha-Sulfofettsäuremethylester von Fettsäuren mit 12 bis 18C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24C-Atomen und 1 bis 6 Doppelbindurgen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22C-Atomen darstellen.

Bevorzugte anionische Tenside sind Alkylsulfate, Alkylpolyglykolethersulfate und Ethercarbonsäuren mit 10 bis 18C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18C-Atomen in der Alkylgruppe. Besonders bevorzugte ampholytische Tenside sind das N-Kokosalkylaminopropionat, das Kokosacylaminoethylaminopropionat und das C₁₂₋₁₈₋Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quatemäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolyinere,
- anionische Polymere wie beispielsweise Polyacrylsäuren, vernetzte Polyacrjrlsäuren, Vinylacetat/Crotonsäure-Copolymere, Vinylpyrrolidon/Vinylacrylat-Copolymere, Vinylacetat/Butylmaleat/lsobomylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein weiterer Gegenstand der vorliegenden Erfindung betrifft die Verwendung von
(a) mindestens einer Verbindung gemäß Formel I und/oder deren physiologisch verträglichen Salze, 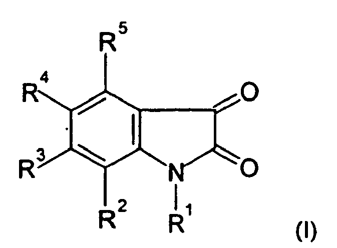 wobei R¹, R², R³, R⁴ und R⁵ wie oben definiert sind,
   gegebenenfalls in Kombination mit mindestens einer Komponente B, die wie oben definiert ist,
   und/oder
(b) mindestens einem Reaktionsprodukt aus der Verbindung gemäß Formel I und der Komponente B
als eine färbende Komponente in Haarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend mindestens eine Verbindung gemäß Formel I und/oder deren physiologisch verträglichen Salze, 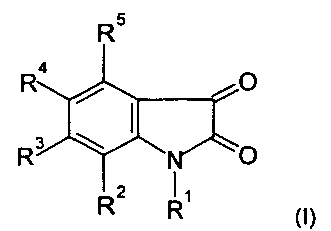 wobei R¹, R², R³, R⁴ und R⁵ wie oben definiert sind,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

In einer weiteren Ausführungsform des erfindungsgemäßen Verfahrens wird neben den Verbindungen der Formel I mindestens eine Verbindung der Komponente B, die wie oben definiert ist, verwendet. Die Kombination aus Isatinderivaten der Formel I und die Verbindungen der Komponente B können entweder gleichzeitig auf das Haar aufgebracht werden oder aber auch nacheinander, d. h. in einem mehrstufigen Verfahren, wobei es unerheblich ist, welche der Komponenten zuerst aufgetragen wird. Die fakultativ enthaltenen Ammonium- oder Metallsalze können dabei den Verbindungen mit der Formel I oder den Verbindungen der Komponente B zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Die Isatinderivate mit der Formel I und die Verbindungen der Komponente B können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

### Beispiele

### Herstellung einer Färbelösung

Es wurde je eine Aufschlämmung bzw. Lösung von 5 mmol des Isatinderivats gemäß Formel I und 5 mmol einer Verbindung gemäß Komponente B in jeweils 25 ml Wasser bei ca. 50°C hergestellt. Wenn das Isatinderivat ohne Komponente B zum Einsatz kommt, werden 50 ml Wasser verwendet. Die Aufschlämmungen wurden nach Abkühlen auf 30°C miteinander vermischt, mit 5 mmol Natriumacetat und einem Tropfen einer 25%igen Fettalkylethersulfat-Lösung versetzt und mit verdünnter NaOH auf einen pH-Wert von pH 9 eingestellt.

In diese Färbemischung wurde bei 30°C 30 Minuten lang eine Strähne zu 90% ergrauten, nicht vorbehandelten Menschenhaares eingebracht. Die Strähne wurde dann 30 Sek. mit lauwarmem Wasser gespült, mit warmer Luft (30°C bis 40°C) getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:
- -: keine oder eine sehr blasse Ausfärbung
- (+): schwache Intensität
- +: mittlere Intensität
- +(+): mittlere bis starke Intensität
- ++: starke intensität
- ++(+): starke bis sehr starke Intensität
- +++: sehr starke Intensität

**Tabelle 1 Ausfärbungen mit Isatinderivaten der Formel I, aeaebenenfalls in Kombination mit Verbindungen der Komponente B**

| **Isatinderivat nach Formel I** | **Komponente B** | **Farbe** | **Intensität** |
|---|---|---|---|
| (1H. 6H)-2,3,7,8-Tetrahydropyrrolo[2',3': 1,2]naphtho[5,6-b]-pyrrol-2,3,7,8-tetron | **-** | grauorange | + |
| (1H,6H)-2,3,7,8-Tetrahydropyrrolo[2',3':1,2]naphtho[5,6-b]-pyrrol-2,3,7,8-tetron | 3-Amino-2-methylamino-6-methoxypyridin | dunkelbraun | +++ |
| (1H, 6H)-2,3,7,8-Tetrahydropyrrolo[2',3':1,2]naphtho[5,6-b]-pyrrol-2,3,7,8-tetron | N,N-Bis(2-hydroxyeth-yl)-p-phenylendiamin H₂SO₄ | graugelb | + |
| (1H, 6H)-2,3,7,8-Tetrahydropyrroto[2',3':1,2]naphtho[5,6-b]-pyrrol-2,3,7,8-tetron | 3-Amino-4-(2-hydroxymethylamino)anisol | graurotbraun | + |
| (1H, 7H)-2,3,5,6-Tetrahydropyrrolo[3,2-f]indol-2,3,5,6-tetron | - | orangegelb | ++ |
| (1H, 7H)-2,3,5,6-Tetrahydropyrrolo[3,2-f]-indol-2,3,5,6-tetron | 2,5-Diaminotoluol H₂SO₄ | braunorange | ++ |
| (1H,7H)-2,3,5,6-Tetrahydropyrrolo[3,2-f]indol-2,3,5,6-tetron | 2,4,5,6-Tetraaminopyrimidin 2 H₂SO₄ | rot | ++(+) |
| (1H,7H)-2,3,5,6-Tetrahydropyrrolo[3,2-f]indol-2,3,5,6-tetron | 1,8-Bis-(2,5-diaminophenoxy)-3,6-dioxaoctan 4 HCl | mittelbraun | ++ |
| (1H, 7H)-2,3,5,6-Tetrahydropyrrolo[3,2-f]indol-2,3,5,6-tetron | 3-Amino-2-methylamino-6-methoxypyridin | dunkelbraun | ++(+) |
| (1H, 7H)-2,3,5,6-Tetrahydropyrrolo[3,2-f]indol-2,3,5,6-tetron | 2-Aminomethyl-4-aminophenol 2 HCl | braunorange | +(+) |
| (1H, 7H)-2,3,5,6-Tetrahydropyrrolo[3,2-f]indol-2,3,5,6-tetron | N,N-Bis(2-hydroxyethyl)-p-phenylendiamin H₂SO₄ | orangebraun | ++ |
| (1H, 7H)-2,3,5,6-Tetrahydropyrroio[3,2-f]lindol-2,3,5,6-tetron | 3-Amino-4-(2-hydroxymethylamino)anisol | gelbbraun | +(+) |

## Patentansprüche

1. Mittel zum Färben von keratinhaltigen Fasern, insbesondere menschlichen Haaren, enthaltend mindestens eine Verbindung gemäß Formel I und/oder deren physiologisch verträglichen Salze 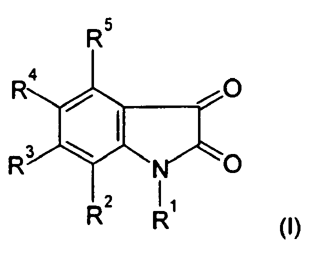 wobei
• R¹ steht für ein Wasserstoffatom, eine C₁-C₄-Alkylgruppe, C₂-C₄-Alkenylgruppe, eine Arylgruppe, eine Aryl-C₁-C₄-alkylgruppe, eine C₁-C₄-Acylgruppe, eine C₁-C₄₋Hydroxyalkylgruppe, eine C₂-C₄-Dihydroxyalkylgruppe, eine C₁-C₄₋Sulfoalkylgruppe, eine C₁-C₄-Carboxyalkylgruppe, eine Gruppe R^{I}R^{II}N-(CH₂)ₘ- oder R^{I}R^{II}R^{III}N⁺-(CH₂)ₘ-, worin m ist gleich 1, 2, 3 oder 4 und R^{I}, R^{II} und R^{III} stehen unabhängig voneinander für ein Wasserstoffatom, eine C₁-C₄₋Alkylgruppe, eine C₁-C₄-Hydroxyalkylgruppe, eine Aryl-C₁-C₄-alkylgruppe oder R^{I} und R^{II} bilden gemeinsam mit dem Stickstoffatom einen heterozyklischen 5-, 6- oder 7-Ring,
oder eine Gruppe gemäß Formel II, 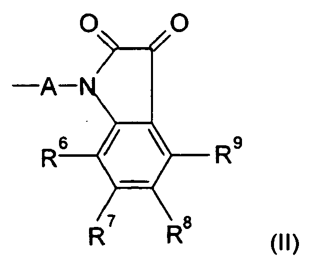 wobei
- A steht für eine C₁-C₈-Alkylengruppe, eine Carbonylgruppe, eine Arylengruppe oder eine Gruppe -(CH₂)ⱼ-O-(CH₂)ₖ-, in der j und k stehen unabhängig voneinenader für eine Zahl 1, 2, 3 oder 4,
- R⁶, R⁷, R⁸ und R⁹ stehen unabhängig voneinander für ein Wasserstoffatom, ein Halogenatom, eine Nitrogruppe, eine Sulfonsäuregruppe, eine Hydroxygruppe, eine Carboxylgruppe, eine C₁-C₄-Alkylgruppe, eine Arylgruppe, eine C₁-C₄-Alkoxygruppe, eine C₁-C₄-Hydroxyalkylgruppe, eine C₂-C₄-Dihydroxyalkylgruppe, eine C₁-C₄-Sulfoalkylgruppe, eine C₁-C₄₋Carboxyalkylgruppe, eine C₁-C₄-Acylgruppe, eine Gruppe R^{IV}R^{V}N-(CH₂)ₙ- oder eine Gruppe R^{IV}R^{V}R^{VI}N⁺-(CH₂)ₙ-, worin R^{IV}, R^{V} und R^{VI} stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe und n ist gleich 1, 2, 3 oder 4, und
• R², R³, R⁴ und R⁵ stehen unabhängig voneinander für ein Wasserstoffatom, ein Halogenatom, eine C₁-C₄-Alkylgruppe, eine Arylgruppe, eine C₁-C₄₋Alkoxygruppe, eine C₁-C₄-Hydroxyalkylgruppe, eine C₂-C₄-Dihydroxyalkylgruppe, eine C₁-C₄-Sulfoalkylgruppe, eine C₁-C₄-Carboxyalkylgruppe, eine C₁-C₄₋Acylgruppe, eine Gruppe R^{VII}R^{VIII}N-(CH₂)_{q}- sowie eine Gruppe R^{VII}R^{VIII}R^{IX}N⁺⁻(CH₂)_{q}-, worin R^{VII}, R^{VIII} und R^{IX} stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe und q ist gleich 1, 2, 3 oder 4, eine Nitrogruppe, eine Sulfonsäuregruppe, eine Hydroxygruppe, eine Carboxylgruppe, eine Gruppe gemäß Formel II, in der A, R⁶, R⁷, R⁸ und R⁹ wie oben definiert sind oder eine Gruppe gemäß Formel III,
wobei zwei benachbarte Reste aus R², R³, R⁴ und R⁵ einen ankondensierten Rest bilden können, der ausgewählt ist aus den Formeln IV bis VIII, 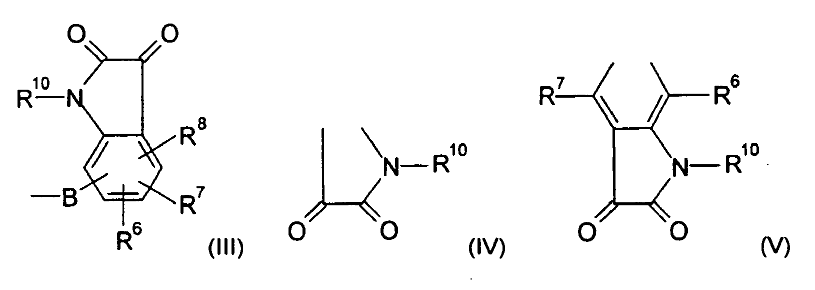 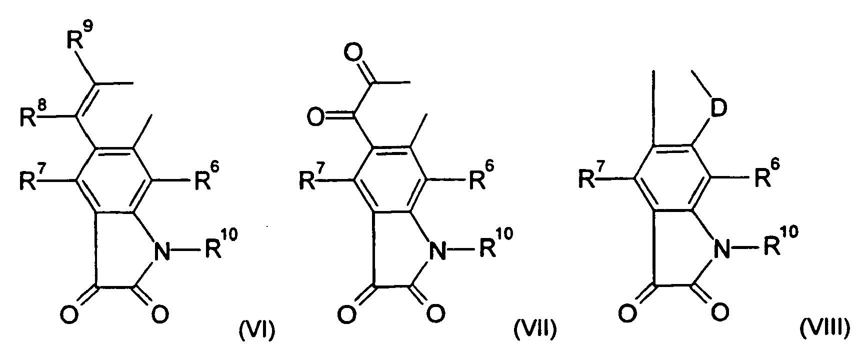 wobei
- R⁶, R⁷, R⁸ und R⁹ wie oben definiert sind,
- R¹⁰ steht für ein Wasserstoffatom, eine C₁-C₄-Alkylgruppe, C₂-C₄₋Alkenylgruppe, eine Arylgruppe, eine Aryl-C₁-C₄-alkylgruppe, eine C₁-C₄₋Acylgruppe, eine C₁-C₄-Hydroxyalkylgruppe, eine C₂-C₄₋Dihydroxyalkylgruppe, eine C₁-C₄-Sulfoalkylgruppe, eine C₁-C₄₋Carboxyalkylgruppe, eine Gruppe R^{X}R^{XI}N-(CH₂)ₛ- oder R^{X}R^{XI}R^{XII}N⁺-(CH₂)ₛ-, worin R^{X}, R^{XI} und R^{XII} stehen unabhängig voneinander für ein Wasserstoffatom, eine C₁-C₄-Alkylgruppe, eine C₁-C₄-Hydroxyalkylgruppe oder eine Aryl-C₁-C₄-alkylgruppe und s ist gleich 1, 2, 3 oder 4,
- B steht für eine direkte Bindung, ein Sauerstoffatom, ein Schwefelatom, eine Disulfidgruppe, eine Gruppe NH, eine Sulfonylgruppe, eine C₁-C₈₋Alkylengruppe, eine Carbonylgruppe, eine Gruppe -O-(CH₂)ₜ-O-, in der t gleich 1, 2, 3 oder 4 ist, eine Gruppe -(CH₂)ᵤ-O-(CH₂)ᵥ-, in der u und v unabhängig voneinander einen Wert von 1, 2, 3 oder 4 haben,
- D steht für eine Methylengruppe, eine Carbonylgruppe und eine Gruppe NH
mit der Maßgabe, daß entweder einer der Reste R¹, R², R³, R⁴ oder R⁵ nach der obigen Definition eine Gruppe der Formeln II oder III ist, oder zwei benachbarte Reste aus R², R³, R⁴ oder R⁵ gemeinsam einen ankondensierten Rest bilden, der ausgewählt ist aus den Formeln IV bis VIII.

2. Mittel nach Anspruch 1, **dadurch gekennzeichnet, daß** zusätzlich als Komponente B mindestens eine Verbindung, ausgewählt aus (a) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, (b) Aminosäuren, (c) CH-aciden Verbindungen.

3. Mittel zum Färben von keratinhaltigen Fasern, insbesondere menschlichen Haaren, enthaltend mindestens ein Reaktionsprodukt als direktziehenden Farbstoff aus einer Verbindung mit der Formel I gemäß Anspruch 1 und einer Verbindung gemäß Amspruch 2.

4. Mittel nach einem der Ansprüche 1 bis 3, **dadurch gekennzeichnet, daß** die Verbindungen mit der Formel I ausgewählt sind aus (1H, 7H)-2,3,5,6-Tetrahydropyrrolo[3,2-f]indol-2,3,5,6-tetron, (1H, 6H)-2,3,7,8-Tetrahydro-pyrrolo[2',3':1,2]-naphtho[5,6-b]pyrrol-2,3,7,8-tetron, (3H, 8H)-1,2,6,7-Tetrahydro-5,10-dichlor-indolo[7,6-g]indol-1,2,6,7-tetron, 5,5'-Oxy-bis(1H-2,3-dihydro-indol-2,3-dion), 6,6'-Oxy-bis(1 H-2,3-dihydro-indol-2,3-dion), 6,6'-Carbonyl-bis-(1H-2,3-dihydro-indol-2,3-dion), (1H, 7H)-2,3,5,6-Tetrahydro-8-chlor-4-methoxy-benzo[1,2-b:5,4-b']dipyrrol-2,3,5,6-tetron, (1H, 8H)-2,3,6,7-Tetrahydro-benzo[2,1-b:3,4-b']dipyrrol-2,3,6,7-tetron, 6,6'-Methylen-bis(1H-2,3-tetrahydro-indol-2,3-dion), 6,6'-Methylen-bis(1H-2,3-tetrahydo-7-chlor-indol-2,3-dion), 1,1'-Methylen-bis(1H-2,3-tetrahydo-indol-2,3-dion), 1,1'-(1,3-Propandiyl)-bis(1H-2,3-tetrahydro-5-methyl-indol-2,3-dion), (1H, 7H)-2,3,5,6-tetrahydro-8-methyl-benzo[1,2-b:5,4-b']dipyrrol-2,3,5,6-tetron, (1H, 1'H)-2,2',3,3' tetrahydro-7,7'-dimethoxy-5,5'-biindol-2,2',3,3'-tetron, (1H, 7H)-2,3,5,6-Tetrahydro-4,8-dimethyl-benzo[1,2-b:5,4-b']dipyrrol-2,3,5,6-tetron, (1H, 1'H)-2,2',3,3'-Tetrahydro-5,5',6,6'-tetrahydroxy-1,1'-dimethyl-4,4'-biindol-2,2',3,3'-tetron.

5. Mittel nach einem der Ansprüche 1 bis 4, **dadurch gekennzeichnet, daß** die Verbindungen der Formel I beziehungsweise die Verbindungen der Komponente B jeweils in einer Menge von 0,03 bis 65 mmol, insbesondere von 1 bis 40 mmol, bezogen auf 100 g des gesamten Färbemittels, enthalten sind.

6. Mittel nach einem der Ansprüche 2 bis 5, **dadurch gekennzeichnet, daß** die primären oder sekundären aromatischen Amine der Komponente B ausgewählt sind aus der Gruppe, die gebildet wird aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)-2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 2,2'-(1,4-Piperazinylen-1,4-dimethylen)-bis-4-aminophenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan, sowie den physiologisch verträglichen Salzen der vorgenannten Verbindungen.

7. Mittel nach einem der Ansprüche 2 bis 6, **dadurch gekennzeichnet, daß** die stickstoffhaltigen heterozyklischen Verbindungen der Komponente B ausgewählt sind der Gruppe, die gebildet wird aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxypyridin, 2,6-Dimethoxy-3,5-diamino-pyridin, 2,4,5-Triamino-pyridin, 2,6-Dihydroxy-3,4-dimethylpyridin, N-[2-(2,4-Diaminophenylamino)-ethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin, 4-Hydroxyindolin und Hydroxypyrimidin-Derivate, sowie den physiologisch verträglichen Salzen der vorgenannten Verbindungen.

8. Mittel nach einem der Ansprüche 2 bis 7, **dadurch gekennzeichnet, daß** die aromatischen Hydroxyverbindungen der Komponente B ausgewählt sind aus der Gruppe, die gebildet wird aus 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure, sowie den physiologisch verträglichen Salzen der vorgenannten Verbindungen.

9. Mittel nach einem der Ansprüche 2 bis 8, **dadurch gekennzeichnet, daß** die Aminosäuren der Komponente B ausgewählt sind aus der Gruppe, die gebildet wird aus Arginin, Histidin, Tyrosin, Phenylalanin, DOPA (Dihydroxyphenylalanin), Ornithin, Prolin, Lysin, Tryptophan, 6-Aminocapronsäure und β-Alanin sowie den physiologisch verträglichen Salzen der vorgenannten Verbindungen.

10. Mittel nach einem der Ansprüche 2 bis 9, **dadurch gekennzeichnet, daß** die CH-aciden Verbindungen der Komponente B ausgewählt sind aus der Gruppe, die gebildet wird aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 1,2-Dimethyl-naphtho[1,2-d]thiazolium-p-toluolsulfonat, 1-Ethyl-2-methyl-naphtho[1,2-d]thiazolium-p-toluolsulfonat, Rhodanin, Rhodanin-3-essigsäure, 1-Methyl-2-chinaldinium-iodid, 1-Methyl-2-chinaldinium-p-toluolsulfonat, 1-Ethyl-2-chinaldiniumiodid, 1-Ethyl-2-chinaldinium-p-toluolsulfonat, 1,4-Dimethylchinoliniumiodid, 1,4-Dimethylchinolinium-p-toluolsulfonat, Barbitursäure, Thiobarbitursäure, 1,3-Dimethylthiobarbitursäure, 1,3-Diethylthiobarbitursäure, Oxindol, 3-Indoxylacetat, 2-Cumaranon, 5-Hydroxy-cumaranon, 6-Hydroxy-cumaranon, 1-Methyl-3-phenyl-2-pyrazolinon, Indan-1,3-dion, Indan-1-on, Benzoylacetonitril, 1-Dicyanmethylenindan, 1,3-Bis(dicyanmethylen)indan, 3-Dicyanmethylenindan-1-on, 1,3-Diiminoisoindolin, 2-Amino-4-imino-1,3-thiazolin-hydrochlorid, 3-Cyan-1,4-dimethyl-6-hydroxy-2-pyridon und 3-Cyan-1-ethyl-6-hydroxy-4-methyl-2-pyridon, sowie den physiologisch verträglichen Salzen der vorgenannten Verbindungen.

11. Mittel nach einem der Ansprüche 2 bis 10, **dadurch gekennzeichnet, daß** die Komponente B ausgewählt ist aus Verbindungen der Gruppe bestehend aus N-(2-Hydroxyethyl)-N-ethyl-p-phenylendiamin, 2-Chlor-p-phenylendiamin, N,N-Bis-(2-hydroxyethyl)-p-phenylendiamin, 2-Aminophenol, 3-Aminophenol, 4-Aminophenol, 2-Amino-6-chlor-4-nitrophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlorphenol, 2-Methyl-5-aminophenol, 3-Methyl-4-aminophenol, 2-Methyl-5-(2-hydroxyethylamino)phenol, 2-Methyl-5-amino-4-chlorphenol, 6-Methyl-3-amino-2-chlorphenol, 2-Aminomethyl-4-aminophenol, 2-Diethylaminomethyl-4-aminophenol, 2-Dimethylaminomethyl-4-aminophenol, 2,6-Dichlor-4-aminophenol, 2-Hydroxymethyl-4-aminophenol, 2,6-Bis(2-hydroxyethylamino)-1-methylbenzol, Bis-(2-hydroxy-5-aminophenyl)methan, Bis-(4,5-amino-2-hydroxyphenyl)-methan, 1,3-Bis(2,4-diaminophenoxy)propan, 1,4-Bis(4-aminophenyl)-1,4-diazacycloheptan, 1,8-Bis(2,5-Diaminophenoxy)-3,6-dioxaoctan, 4,4'-Diaminodiphenylamin, 3,4-Methylendioxyphenol, 3,4-Diaminobenzoesäure, 2,5-Diaminopyridin, 2-Dimethylamino-5-aminopyridin, 2-Amino-3-hydroxypyridin, 3-Amino-2-methylamino-6-methoxypyridin, 2,3-Diamino-6-metnoxypyridin, 3,5-Diamino-2,6-dimethoxypyridin, 2,6-Dihydroxy-3,4-dimethylpyridin, 2-Hydroxy-4,5,6-triaminopyrimidin, 4-Hydroxy-2,5,6-triaminopyrimidin, 2,4,5,6-Tetraaminopyrimidin, 2-Methylamino-4,5,6-triamino-pyrimidin, 3,5-Diaminopyrazol, 3-Amino-5-hydroxypyrazol, 4,5-Diamino-1-(2-hydroxyethyl)-pyrazol, 5,6-Dihydroxyindol, 5,6-Dihydroxyindolin, 4-Amino-2,3-dimethyl-1-phenyl-3-pyrazolin-5-on (4-Aminoantipyrin), 2,3-Dimethylbenzothiazolium-p-toluolsulfonat, 1,2,3,3-Tetramethyl-3H-indolinium-p-toluolsulfonat, Thiobarbitursäure, Rhodanin, 2,3-Dimethyl-benzothiazolium-p-toluolsulfonat, 1,2-Dimethyl-naphtho[1,2-d]thiazolium-p-toluolsulfonat, 1-Methyl-2-chinaldinium-p-toluolsulfonat, 1,4-Dimethylchinolinium-p-toluolsulfonat, β-Alanin, L-Prolin, L-Lysin, DL-Tyrosin sowie jeweils aus den vorzugsweise mit anorganischen Säuren gebildeten physiologisch verträglichen Salzen dieser Verbindungen.

12. Mittel nach einem der Ansprüche 1 bis 11, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

13. Mittel nach einem der Ansprüche 1 bis 12, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

14. Mittel nach einem der Ansprüche 1 bis 13, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

15. Mittel nach einem der Ansprüche 1 bis 14, **dadurch gekennzeichnet, daß** es Oxidationsmittel, insbesondere H₂O₂, in einer Menge von 0,01 bis 6 Gew.-%, bezogen auf die Anwendungslösung, enthält.

16. Mittel nach einem der Ansprüche 1 bis 15, **dadurch gekennzeichnet, daß** es anionische, zwitterionische oder nichtionische Tenside enthält.

17. Verwendung von
(a) mindestens einer Verbindung gemäß Anspruch 1, gegebenenfalls in Kombination mit mindestens einer Komponente B gemäß Anspruch 2,
und/oder
(b) mindestens einem Reaktionsprodukt aus der Verbindung gemäß Anspruch 1 und der Komponente B
als eine färbende Komponente in Haarfärbemitteln.

18. Verfahren zum Färben von keratinhaltigen Fasern, insbesondere menschlichen Haaren, worin ein Färbemittel, enthaltend eine Verbindung gemäß Anspruch 1, sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

19. Verfahren gemäß Anspruch 18, **dadurch gekennzeichnet, daß** zusätzlich eine Komponente B, enthaltend mindestens eine Verbindung ausgewählt aus (a) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, (b) Aminosäuren, (c) CH-aciden Verbindungen, sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Agent for dyeing keratinous fibres, in particular human hair, comprising at least one compound according to formula I and/or physiologically compatible salts thereof 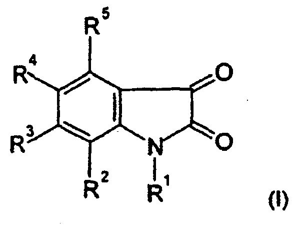 where
• R¹ is a hydrogen atom, a C₁-C₄-alkyl group, C₂-C₄₋alkenyl group, an aryl group, an aryl-C₁-C₄-alkyl group, a C₁-C₄-acyl group, a C₁-C₄-hydroxyalkyl group, a C₂-C₄-dihydroxyalkyl group, a C₁-C₄₋sulphoalkyl group, a C₁-C₄-carboxyalkyl group, a group R^{I}R^{II}N-(CH₂)ₘ- or R^{I}R^{II}R^{III}N⁺-(CH₂)ₘ-, in which m is 1, 2, 3 or 4 and R^{I}, R^{II} and R^{III}, independently of one another, are a hydrogen atom, a C₁-C₄-alkyl group, a C₁-C₄-hydroxyalkyl group, an aryl-C₁-C₄₋alkyl group or R^{I} and R^{II} together with the nitrogen atom form a heterocyclic 5-, 6- or 7- membered ring,
or a group according to formula II, 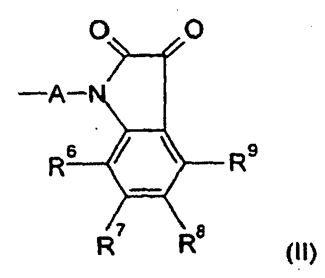
where
- A is a C₁-C₈-alkylene group, a carbonyl group, an arylene group or a group -(CH₂)ⱼ-O-(CH₂)ₖ-, in which j and k, independently of one another, are a number 1, 2, 3 or 4,
- R⁶, R⁷, R⁸ and R⁹, independently of one another, are a hydrogen atom, a halogen atom, a nitro group, a sulphonic acid group, a hydroxy group, a carboxyl group, a C₁-C₄-alkyl group, an aryl group, a C₁-C₄-alkoxy group, a C₁-C₄-hydroxyalkyl group, a C₂-C₄-dihydroxyalkyl group, a C₁-C₄₋sulphoalkyl group, a C₁-C₄-carboxyalkyl group, a C₁-C₄-acyl group, a group R^{IV}R^{V}N-(CH₂)ₙ- or a group R^{IV}R^{V}R^{VI}N⁺-(CH₂)ₙ-, in which R^{IV}, R^{V} and R^{VI}, independently of one another, are a hydrogen atom, a C₁-C₄-alkyl group, a C₁-C₄-hydroxyalkyl group or an aryl-C₁-C₄-alkyl group and n is 1, 2, 3 or 4, and
- R², R³, R⁴ and R⁵, independently of one another, are a hydrogen atom, a halogen atom, a C₁-C₄-alkyl group, an aryl group, a C₁-C₄-alkoxy group, a C₁-C₄₋hydroxyalkyl group, a C₂-C₄-dihydroxyalkyl group, a C₁-C₄-sulphoalkyl group, a C₁-C₄-carboxyalkyl group, a C₁-C₄-acyl group, a group R^{VII}R^{VIII}N-(CH₂)_{q}- and a group R^{VII}R^{VIII}R^{IX}N⁺-(CH₂)_{q}-, in which R^{VII}, R^{VIII} and R^{IX}, independently of one another, are a hydrogen atom, a C₁-C₄-alkyl group, a C₁-C₄-hydroxyalkyl group or an aryl-C₁-C₄-alkyl group and q is 1, 2, 3 or 4, a nitro group, a sulphonic acid group, a hydroxy group, a carboxyl group, a group according to formula II in which A, R⁶, R⁷, R⁸ and R⁹ are as defined above or a group according to formula III,
where two adjacent radicals from R², R³, R⁴ and R⁵ can form a fused-on radical which is chosen from the formulae IV to VIII, 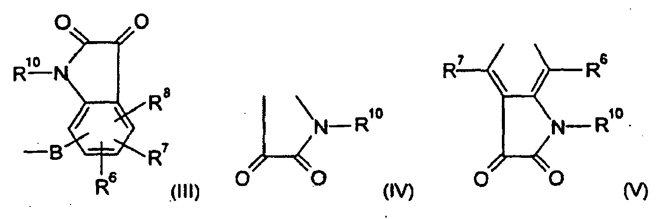 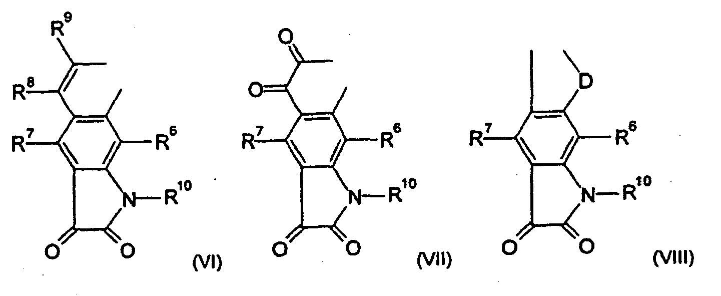 where
- R⁶, R⁷, R⁸ and R⁹ are as defined above,
- R¹⁰ is a hydrogen atom, a C₁-C₄-alkyl group, C₂-C₄-alkenyl group, an aryl group, an aryl-C₁-C₄-alkyl group, a C₁-C₄-acyl group, a C₁-C₄₋hydroxyalkyl group, a C₂-C₄-dihydroxyalkyl group, a C₁-C₄-sulphoalkyl group, a C₁-C₄₋carboxyalkyl group, a group R^{X}R^{XI}N-(CH₂)ₛ- or R^{X}R^{XI}R^{XII}N⁺-(CH₂)ₛ-, in which R^{X}, R^{XI} and R^{XII}, independently of one another, are a hydrogen atom, a C₁-C₄-alkyl group, a C₁-C₄-hydroxyalkyl group or an aryl-C₁-C₄-alkyl group and s is 1, 2, 3 or 4,
- B is a direct bond, an oxygen atom, a sulphur atom, a disulphide group, a group NH, a sulphonyl group, a C₁-C₈-alkylene group, a carbonyl group, a group -O-(CH₂)ₜ-O-, in which t is 1, 2, 3 or 4, a group -(CH₂)ᵤ-O-(CH₂)ᵥ-, in which u and v, independently of one another, have a value of 1, 2, 3 or 4,
- D is a methylene group, a carbonyl group or a group NH
with the proviso that either one of the radicals R¹, R², R³, R⁴ or R⁵ according to the above definition is a group of the formulae II or III, or two adjacent radicals from R², R³, R⁴ or R⁵ together form a fused-on radical which is chosen from the formulae IV to VIII.

2. Agent according to Claim 1, **characterized in that** additionally, as component B, at least one compound chosen from (a) compounds with primary or secondary amino or hydroxy group, chosen from aromatic hydroxy compounds, primary or secondary aromatic amines and nitrogen-containing heterocyclic compounds, (b) amino acids, (c) CH acidic compounds is present.

3. Agent for dyeing keratinous fibres, in particular human hair, comprising at least one reaction product as direct dye from a compound with the formula I according to Claim 1 and a compound according to Claim 2.

4. Agent according to one of Claims 1 to 3, **characterized in that** the compounds with the formula I are chosen from (1H,7H)-2,3,5,6-tetrahydropyrrolo[3,2-f]indole-2,3,5,6-tetrone, (1H,6H)-2,3,7,8-tetrahydropyrrolo[2',3':1,2]naphtho[5,6-b]pyrrole-2,3,7,8-tetrone, (3H,8H)-1,2,6,7-tetrahydro-5,10-dichloro-indolo[7,6-g]indole-1,2,6,7-tetrone, 5,5'-oxybis(1H-2,3-dihydroindole-2,3-dione), 6,6'-oxybis(1H-2,3-dihydroindole-2,3-dione), 6,6'-carbonylbis(1H-2,3-dihydroindole-2,3-dione), (1H,7H)-2,3,5,6-tetrahydro-8-chloro-4-methoxybenzo[1,2-b:5,4-b']dipyrrole-2,3,5,6-tetrone, (1H,8H)-2,3,6,7-tetrahydrobenzo[2,1-b:3,4-b']dipyrrole-2,3,6,7-tetrone, 6,6'-methylenebis(1H-2,3-tetrahydroindole-2,3-dione), 6,6'-methylenebis(1H-2,3-tetrahydro-7-chloroindole-2,3-dione), 1,1'-methylene-bis(1H-2,3-tetrahydroindole-2,3-dione), 1,1'-(1,3-propanediyl)bis(1H-2,3-tetrahydro-5-methylindole-2,3-dione), (1H,7H)-2,3,5,6-tetrahydro-8-methylbenzo[1,2-b:5,4-b']dipyrrole-2,3,5,6-tetrone, (1H,1'H)-2,2',3,3'-tetrahydro-7,7'-dimethoxy-5,5'-biindole-2,2',3,3'-tetrone, (1H,7H)-2,3,5,6-tetrahydro-4,8-dimethylbenzo[1,2-b:5,4-b']dipyrrole-2,3,5,6-tetrone, (1H,1'H)-2,2',3,3'-tetrahydro-5,5',6,6'-tetrahydroxy-1,1'-dimethyl-4,4'-biindole-2,2',3,3'-tetrone.

5. Agent according to one of Claims 1 to 4, **characterized in that** the compounds of the formula I or the compounds of component B are each present in an amount of from 0.03 to 65 mmol, in particular from 1 to 40 mmol, based on 100 g of the total dyeing agent.

6. Agent according to one of Claims 2 to 5, **characterized in that** the primary or secondary aromatic amines of component B are chosen from the group which is formed from N,N-dimethyl-p-phenylenediamine, N,N-diethyl-p-phenylenediamine, N-(2-hydroxyethyl)-N-ethyl-p-phenylenediamine, N,N-bis(2-hydroxyethyl)-p-phenylenediamine, N-(2-methoxyethyl)-p-phenylenediamine, 2,3-dichloro-p-phenylenediamine, 2,4-dichloro-p-phenylenediamine, 2,5-dichloro-p-phenylenediamine, 2-chloro-p-phenylenediamine, 2,5-dihydroxy-4-morpholino-aniline, 2-aminophenol, 3-aminophenol, 4-aminophenol, 2-aminomethyl-4-aminophenol, 2-hydroxymethyl-4-aminophenol, o-phenylenediamine, m-phenylenediamine, p-phenylenediamine, 2,5-diaminotoluene, 2,5-diaminophenol, 2,5-diaminoanisole, 2,5-diaminophenetol, 4-amino-3-methylphenol, 2-(2,5-diaminophenyl)ethanol, 2,4-diaminophenoxyethanol, 2-(2,5-diaminophenoxy)-ethanol, 3-amino-4-(2-hydroxyethyloxy)phenol, 3,4-methylenedioxyphenol, 3,4-methylenedioxyaniline, 3-amino-2,4-dichlorophenol, 4-methylaminophenol, 2-methyl-5-aminophenol, 3-methyl-4-aminophenol, 2-methyl-5-(2-hydroxyethylamino)phenol, 3-amino-2-chloro-6-methylphenol, 2-methyl-5-amino-4-chlorophenol, 5-(2-hydroxyethylamino)-4-methoxy-2-methylphenol; 4-amino-2-hydroxymethylphenol, 2-(diethylaminomethyl)-4-aminophenol, 4-amino-1-hydroxy-2-(2-hydroxyethylaminomethyl)benzene, 1-hydroxy-2-amino-5-methylbenzene, 1-hydroxy-2-amino-6-methylbenzene, 2-amino-5-acetamidophenol, 1,3-dimethyl-2,5-diaminobenzene, 5-(3-hydroxypropylamino)-2-methylphenol, 5-amino-4-methoxy-2-methylphenol, N,N-dimethyl-3-aminophenol, N-cyclopentyl-3-aminophenol, 5-amino-4-fluoro-2-methylphenol, 2,4-diamino-5-fluorotoluene, 2,4-diamino-5-(2-hydroxyethoxy)toluene, 2,4-diamino-5-methylphenetol, 3,5-diamino-2-methoxy-1-methylbenzene, 2-amino-4-(2-hydroxyethylamino)anisole, 2,6-bis(2-hydroxyethylamino)-1-methylbenzene, 1,3-diamino-2,4-dimethoxybenzene, 3,5-diamino-2-methoxytoluene, 2-aminobenzoic acid, 3-aminobenzoic acid, 4-aminobenzoic acid, 2-aminophenylacetic acid, 3-aminophenylacetic acid, 4-aminophenylacetic acid, 2,3-diaminobenzoic acid, 2,4-diaminobenzoic acid, 2,5-diaminobenzoic acid, 3,4-diaminobenzoic acid, 3,5-diaminobenzoic acid, 4-aminosalicylic acid, 5-aminosalicylic acid, 3-amino-4-hydroxybenzoic acid, 4-amino-3-hydroxybenzoic acid, 2-aminobenzenesulphonic acid, 3-aminobenzenesulphonic acid, 4-aminobenzenesulphonic acid, 3-amino-4-hydroxybenzenesulphonic acid, 4-amino-3-hydroxynaphthalene-1-sulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-triaminobenzene, 1,2,4-triaminobenzene, 1,2,4,5-tetraaminobenzene, 2,4,5-triaminophenol, pentaaminobenzene, hexaaminobenzene, 2,4,6-triaminoresorcinol, 4,5-diaminopyrocatechin, 4,6-diaminopyrogallol, 1-(2-hydroxy-5-aminobenzyl)-2-imidazolidinone, 2,2'-(1,4-piperazinylene-1,4-dimethylene)bis-4-aminophenol, 3,5-diamino-4-hydroxypyrocatechin, 1,4-bis(4-aminophenyl)-1,4-diazacycloheptane, aromatic nitriles, such as 2-amino-4-hydroxybenzonitrile, 4-amino-2-hydroxybenzonitrile, 4-aminobenzonitrile, 2,4-diaminobenzonitrile, nitro-group-containing amino compounds, such as 3-amino-6-methylamino-2-nitropyridine, picramic acid, [8-[(4-amino-2-nitrophenyl)azo]-7-hydroxynaphth-2-yl]trimethylammonium chloride, [8-((4-amino-3-nitrophenyl)azo)-7-hydroxynaphth-2-yl]trimethylammonium chloride (Basic Brown 17), 1-hydroxy-2-amino-4,6-dinitrobenzene, 1-amino-2-nitro-4-[bis(2-hydroxyethyl)-amino]benzene, 1-amino-2-[(2-hydroxyethyl)amino]-5-nitrobenzene (HC Yellow No. 5), 1-amino-2-nitro-4-[(2-hydroxyethyl)amino]benzene (HC Red No. 7), 2-chloro-5-nitro-N-2-hydroxyethyl-1,4-phenylenediamine, 1-[(2-hydroxyethyl)amino]-2-nitro-4-aminobenzene (HC Red No. 3), 4-amino-3-nitrophenol, 4-amino-2-nitrophenol, 6-nitro-o-toluidine, 1-amino-3-methyl-4-[(2-hydroxyethyl)amino]-6-nitrobenzene (HC Violet No. 1), 1-amino-2-nitro-4-[(2,3-dihydroxypropyl)amino]-5-chlorobenzene (HC Red No. 10), 2-(4-amino-2-nitroanilino)benzoic acid, 6-nitro-2,5-diaminopyridine, 2-amino-6-chloro-4-nitrophenol, 1-amino-2-(3-nitrophenylazo)-7-phenylazo-8-naphthol-3,6-disulphonic acid disodium salt (Acid Blue No. 29), 1-amino-2-(2-hydroxy-4-nitrophenylazo)-8-naphthol-3,6-disulphonic acid disodium salt (Palatine Chrome Green), 1-amino-2-(3-chloro-2-hydroxy-5-nitrophenylazo)-8-naphthol-3,6-disulphonic acid disodium salt (Gallion), 4-amino-4'-nitrostilbene-2,2'-disulphonic acid disodium salt, 2,4-diamino-3',5'-dinitro-2'-hydroxy-5-methylazobenzene (Mordant Brown 4), 4'-amino-4-nitrodiphenylamine-2-sulphonic acid, 4'-amino-3'-nitrobenzophenone-2-carboxylic acid, 1-amino-4-nitro-2-(2-nitrobenzylideneamino)benzene, 2-[2-(diethylamino)ethylamino]-5-nitroaniline, 3-amino-4-hydroxy-5-nitrobenzenesulphonic acid, 3-amino-3'-nitrobiphenyl, 3-amino-4-nitroacenaphthene, 2-amino-1-nitronaphthalene, 5-amino-6-nitrobenzo-1,3-dioxole, anilines, in particular nitro-group-containing anilines, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)-benzene, 2-nitro-1-amino-4-[bis(2-hydroxyethyl)amino]-benzene, 4-amino-2-nitrodiphenylamine-2'-carboxylic acid, 1-amino-5-chloro-4-(2-hydroxyethylamino)-2-nitrobenzene, 4,4'-diaminostilbene and its hydrochloride, 4,4'-diaminostilbene-2,2'-disulphonic acid mono- or di-Na salt, 4-amino-4'-dimethylaminostilbene and its hydrochloride, 4,4'-diaminodiphenylmethane, 4,4'-diaminodiphenyl sulphide, 4,4'-diaminodiphenyl sulphoxide, 4,4'-diaminodiphenylamine, 4,4'-diaminodiphenylamine-2-sulphonic acid, 4,4'-diaminobenzophenone, 4,4'-diaminodiphenyl ether, 3,3',4,4'-tetraaminodiphenyl, 3,3',4,4'-tetraaminobenzophenone, 1,3-bis(2,4-diaminophenoxy)propane, 1,8-bis(2,5-diaminophenoxy)-3,6-dioxaoctane, 1,3-bis(4-aminophenylamino)propane, 1,3-bis(4-aminophenylamino)-2-propanol, 1,3-bis[N-(4-aminophenyl)-2-hydroxyethylamino]-2-propanol, N,N-bis[2-(4-aminophenoxy)ethyl]methylamine, N-phenyl-1,4-phenylenediamine and bis(5-amino-2-hydroxyphenyl)methane, and the physiologically compatible salts of the abovementioned compounds.

7. Agent according to one of Claims 2 to 6, **characterized in that** the nitrogen-containing heterocyclic compounds of component B are chosen from the group which is formed from 2-aminopyridine, 3-aminopyridine, 4-aminopyridine, 2-amino-3-hydroxypyridine, 2,6-diaminopyridine, 2,5-diaminopyridine, 2-(aminoethylamino)-5-aminopyridine, 2,3-diaminopyridine, 2-dimethylamino-5-aminopyridine, 2-methylamino-3-amino-6-methoxypyridine, 2,3-diamino-6-methoxypyridine, 2,6-dimethoxy-3,5-diaminopyridine, 2,4,5-triaminopyridine, 2,6-dihydroxy-3,4-dimethylpyridine, N-[2-(2,4-diaminophenylamino)ethyl]-N-(5-amino-2-pyridyl)amine, N-[2-(4-aminophenyl)aminoethyl]-N-(5-amino-2-pyridyl)amine, 2,4-dihydroxy-5,6-diaminopyrimidine, 4,5,6-triaminopyrimidine, 4-hydroxy-2,5,6-triaminopyrimidine, 2-hydroxy-4,5,6-triaminopyrimidine, 2,4,5,6-tetraaminopyrimidine, 2-methylamino-4,5,6-triaminopyrimidine, 2,4-diaminopyrimidine, 4,5-diaminopyrimidine, 2-amino-4-methoxy-6-methylpyrimidine, 3,5-diaminopyrazole, 3,5-diamino-1,2,4-triazole, 3-aminopyrazole, 3-amino-5-hydroxypyrazole, 1-phenyl-4,5-diaminopyrazole, 1-(2-hydroxyethyl)-4,5-diaminopyrazole, 1-phenyl-3-methyl-4,5-diaminopyrazole, 4-amino-2,3-dimethyl-1-phenyl-3-pyrazolin-5-one (4-aminoantipyrine), 1-phenyl-3-methypyrazol-5-one, 2-aminoquinoline, 3-aminoquinoline, 8-aminoquinoline, 4-aminoquinaldine, 2-aminonicotinic acid, 6-aminonicotinic acid, 5-aminoisoquinoline, 5-aminoindazole, 6-aminoindazole, 5-aminobenzimidazole, 7-aminobenzimidazole, 5-aminobenzothiazole, 7-aminobenzothiazole, 2,5-dihydroxy-4-morpholinoaniline, and indole and indoline derivatives, such as 4-aminoindole, 5-aminoindole, 6-aminoindole, 7-aminoindole, 5,6-dihydroxyindole, 5,6-dihydroxyindoline, 4-hydroxyindoline and hydroxypyrimidine derivatives, and the physiologically compatible salts of the abovementioned compounds.

8. Agent according to one of Claims 2 to 7, **characterized in that** the aromatic hydroxy compounds of component B are chosen from the group which is formed from 2-methylresorcinol, 4-methylresorcinol, 5-methylresorcinol, 2,5-dimethylresorcinol, resorcinol, 3-methoxyphenol, pyrocatechin, hydroquinone, pyrogallol, phloroglucinol, hydroxyhydroquinone, 2-methoxyphenol, 3-methoxyphenol, 4-methoxyphenol, 3-dimethylaminophenol, 2-(2-hydroxyethyl)phenol, 3,4-methylenedioxyphenol, 2,4-dihydroxybenzoic acid, 3,4-dihydroxybenzoic acid, 2,4-dihydroxyphenylacetic acid, 3,4-dihydroxyphenylacetic acid, gallic acid, 2,4,6-trihydroxybenzoic acid, 2,4,6-trihydroxyacetophenone, 2-chlororesorcinol, 4-chlororesorcinol, 1-naphthol, 1,5-dihydroxynaphthalene, 2,3-dihydroxynaphthalene, 2,7-dihydroxynaphthalene, 6-dimethylamino-4-hydroxy-2-naphthalenesulphonic acid and 3,6-dihydroxy-2,7-naphthalenesulphonic acid, and the physiologically compatible salts of the abovementioned compounds.

9. Agent according to one of Claims 2 to 8, **characterized in that** the amino acids of component B are chosen from the group which is formed from arginine, histidine, tyrosine, phenylalanine, DOPA (dihydroxyphenylalanine), ornithine, proline, lysine, tryptophan, 6-aminocaproic acid and β-alanine, and the physiologically compatible salts of the abovementioned compounds.

10. Agent according to one of Claims 2 to 9, **characterized in that** the CH-acidic compounds of component B are chosen from the group which is formed from 1,2,3,3-tetramethyl-3H-indolium iodide, 1,2,3,3-tetramethyl-3H-indolium p-toluenesulphonate, 1,2,3,3-tetramethyl-3H-indolium methanesulphonate, 1,3,3-trimethyl-2-methyleneindoline (Fischer's base), 2,3-dimethylbenzothiazolium iodide, 2,3-dimethylbenzothiazolium p-toluene sulphonate, 1,2-dimethylnaphtho[1,2-d]thiazolium p-toluenesulphonate, 1-ethyl-2-methylnaphtho[1,2-d]thiazolium p-toluenesulphonate, rhodanine, rhodanine-3-acetic acid, 1-methyl-2-quinaldinium iodide, 1-methyl-2-quinaldinium p-toluenesulphonate, 1-ethyl-2-quinaldinium iodide, 1-ethyl-2-quinaldinium p-toluenesulphonate, 1,4-dimethylquinolinium iodide, 1,4-dimethylquinolinium p-toluenesulphonate, barbituric acid, thiobarbituric acid, 1,3-dimethylthiobarbituric acid, 1,3-diethylthiobarbituric acid, oxindole, 3-indoxyl acetate, 2-coumaranone, 5-hydroxycoumaranone, 6-hydroxycoumaranone, 1-methyl-3-phenyl-2-pyrazolinone, indane-1,3-dione, indan-1-one, benzylacetonitrile, 1-dicyanomethyleneindane, 1,3-bis(dicyanomethylene)indane, 3-dicyanomethyleneindan-1-one, 1,3-diiminoisoindoline, 2-amino-4-imino-1,3-thiazoline hydrochloride, 3-cyano-1,4-dimethyl-6-hydroxy-2-pyridone and 3-cyano-1-ethyl-6-hydroxy-4-methyl-2-pyridone, and the physiologically compatible salts of the abovementioned compounds.

11. Agent according to one of Claims 2 to 10, **characterized in that** component B is chosen from compounds of the group consisting of N-(2-hydroxyethyl)-N-ethyl-p-phenylenediamine, 2-chloro-p-phenylenediamine, N,N-bis(2-hydroxyethyl)-p-phenylenediamine, 2-aminophenol, 3-aminophenol, 4-aminophenol, 2-amino-6-chloro-4-nitrophenol, p-phenylenediamine, 2-(2,5-diaminophenyl)ethanol, 2,5-diaminotoluene, 3,4-methylenedioxyaniline, 2-amino-4-(2-hydroxyethylamino)anisole, 2-(2,4-diaminophenoxy)ethanol, 3-amino-2,4-dichlorophenol, 2-methyl-5-aminophenol, 3-methyl-4-aminophenol, 2-methyl-5-(2-hydroxyethylamino)phenol, 2-methyl-5-amino-4-chlorophenol, 6-methyl-3-amino-2-chlorophenol, 2-aminomethyl-4-aminophenol, 2-diethylaminomethyl-4-aminophenol, 2-dimethylaminomethyl-4-aminophenol, 2,6-dichloro-4-aminophenol, 2-hydroxymethyl-4-aminophenol, 2,6-bis(2-hydroxyethylamino)-1-methylbenzene, bis(2-hydroxy-5-aminophenyl)methane, bis(4,5-amino-2-hydroxyphenyl)methane, 1,3-bis(2,4-diaminophenoxy)propane, 1,4-bis(4-aminophenyl)-1,4-diazacycloheptane, 1,8-bis(2,5-diaminophenoxy)-3,6-dioxaoctane, 4,4'-diaminodiphenylamine, 3,4-methylenedioxyphenol, 3,4-diaminobenzoic acid, 2,5-diaminopyridine, 2-dimethylamino-5-aminopyridine, 2-amino-3-hydroxypyridine, 3-amino-2-methylamino-6-methoxypyridine, 2,3-diamino-6-methoxypyridine, 3,5-diamino-2,6-dimethoxypyridine, 2,6-dihydroxy-3,4-dimethylpyridine, 2-hydroxy-4,5,6-triaminopyrimidine, 4-hydroxy-2,5,6-triaminopyrimidine, 2,4,5,6-tetraaminopyrimidine, 2-methylamino-4,5,6-triaminopyrimidine, 3,5-diaminopyrazole, 3-amino-5-hydroxypyrazole, 4,5-diamino-1-(2-hydroxyethyl)pyrazole, 5,6-dihydroxyindole, 5,6-dihydroxyindoline, 4-amino-2,3-dimethyl-1-phenyl-3-pyrazolin-5-one (4-aminoantipyrine), 2,3-dimethylbenzothiazolium p-toluenesulphonate, 1,2,3,3-tetramethyl-3H-indolinium p-toluenesulphonate, thiobarbituric acid, rhodanine, 2,3-dimethylbenzothiazolium p-toluenesulphonate, 1,2-dimethylnaphtho[1,2-d]thiazolium p-toluenesulphonate, 1-methyl-2-quinaldinium p-toluenesulphonate, 1,4-dimethylquinolinium p-toluenesulphonate, β-alanine, L-proline, L-lysine, DL-tyrosine, and in each case from the physiologically compatible salts of these compounds formed preferably with inorganic acids.

12. Agent according to one of Claims 1 to 11, **characterized in that** it comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, histidine, pyrrolidine, proline, pyrrolidone, pyrrolidone-5-carboxylic acid, pyrazole, 1,2,4-triazole, piperazidine or any mixtures thereof.

13. Agent according to one of Claims 1 to 12, **characterized in that** it comprises direct dyes from the group of nitrophenylenediamines, nitroaminophenols, anthraquinones or indophenols, preferably in an amount of from 0.01 to 20% by weight, based on the total dyeing agent.

14. Agent according to one of Claims 1 to 13, **characterized in that** ammonium or metal salts chosen from the group of formates, carbonates, halides, sulphates, butyrates, valeriates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc, are added.

15. Agent according to one of Claims 1 to 14, **characterized in that** it comprises oxidizing agents, in particular H₂O₂, in an amount of from 0.01 to 6% by weight, based on the application solution.

16. Agent according to one of Claims 1 to 15, **characterized in that** it comprises anionic, zwitterionic or nonionic surfactants.

17. Use of
(a) at least one compound according to Claim 1, optionally in combination with at least one component B according to Claim 2,
and/or
(b) at least one reaction product from the compound according to Claim 1 and component B
as a dyeing component in hair dyeing agents.

18. Method of dyeing keratinous fibres, in particular human hair, in which a dyeing agent comprising a compound according to Claim 1, and also customary cosmetic ingredients, is applied to the keratinous fibres, left on the fibres for a certain amount of time, usually about 30 minutes, and then rinsed out again or washed out with a shampoo.

19. Method according to Claim 18, **characterized in that** additionally a component B comprising at least one compound chosen from (a) compounds with primary or secondary amino or hydroxy group, chosen from aromatic hydroxy compounds, primary or secondary aromatic amines and nitrogen-containing heterocyclic compounds, (b) amino acids, (c) CH-acidic compounds, and customary cosmetic ingredients, is applied to the keratinous fibres, left on the fibres for a certain amount of time, usually about 30 minutes, and then rinsed out again or washed out with a shampoo.

## Revendications

1. Agent de teinture pour fibres kératiniques, en particulier de cheveux humains, contenant au moins un composé de formule I et/ou un de ses sels physiologiquement acceptable 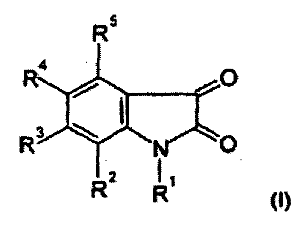 dans laquelle
• R¹ représente un atome d'hydrogène, un groupe alkyle en C₁-C₄, un groupe alcényle en C₂-C₄, un groupe aryle, un groupe aryl(alkyle en C₁-C₄), un groupe acyle en C₁-C₄, un groupe hydroxy(alkyle en C₁₋C₄), un groupe dihydroxy(alkyle en C₂-C₄), un groupe sulfo(alkyle en C₁-C₄), un groupe carboxy(alkyle en C₁-C₄), un groupe R^{I}R^{II}N-(CH₂)ₘ- ou R^{I}R^{II}R^{III}N⁺-(CH₂)ₘ-, où m est égal à 1, 2, 3 ou 4 et R^{I}, R^{II} et R^{III} représentent indépendamment l'un de l'autre un atome d'hydrogène, un groupe alkyle en C₁-C₄, un groupe hydroxy(alkyle en C₁-C₄), un groupe aryl(alkyle en C₁-C₄) ou R^{I} et R^{II} forment conjointement avec l'atome d'azote un hétérocycle à 5, 6 ou 7 maillons,
ou un groupe de formule II, 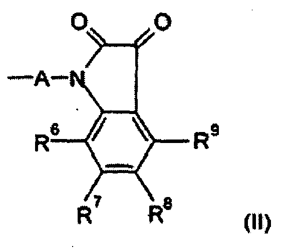 dans laquelle
- A représente un groupe alkylène en C₁-C₈, un groupe carbonyle, un groupe arylène ou un groupe -(CH₂)ⱼ-O-(CH₂)ₖ-, où j et k représentent indépendamment l'un de l'autre un nombre égal à 1,2,3ou4,
- R⁶, R⁷, R⁸ et R⁹ représentent indépendamment l'un de l'autre un atome d'hydrogène, un atome d'halogène, un groupe nitro, un groupe acide sulfonique, un groupe hydroxy, un groupe carboxyle, un groupe alkyle en C₁-C₄, un groupe aryle, un groupe alkoxy en C₁-C₄, un groupe hydroxy(alkyle en C₁-C₄), un groupe dihydroxy(alkyle en C₂-C₄), un groupe sulfo(alkyle en C₁₋C₄), un groupe carboxy(alkyle en C₁-C₄), un groupe acyle en C₁₋C₄, un groupe R^{IV}R^{V}N-(CH₂)ₙ- ou un groupe R^{IV}R^{V}R^{VI}N⁺-(CH₂)ₙ-, où R^{IV}, R^{V} et R^{VI} représentent indépendamment l'un de l'autre un atome d'hydrogène, un groupe alkyle en C₁-C₄, un groupe hydroxy(alkyle en C₁-C₄), ou un groupe aryl(alkyle en C₁-C₄), et n est égal à 1,2,3 ou 4, et
• R², R³, R⁴ et R⁵ représentent indépendamment l'un de l'autre un atome d'hydrogène, un atome d'halogène, un groupe alkyle en C₁-C₄, un groupe aryle, un groupe alcoxy en C₁-C₄, un groupe hydroxy(alkyle en C₁-C₄), un groupe dihydroxy(alkyle en C₂-C₄), un groupe sulfo(alkyle en C₁-C₄), un groupe carboxy(alkyle en C₁-C₄), un groupe acyle en C₁-C₄, un groupe R^{VII}R^{VIII}N-(CH₂)_{q}- ainsi qu'un groupe R^{VII}R^{VIII}R^{IX}N⁺-(CH₂)_{q}-, où R^{VII}, R^{VIII} et R^{IX} représentent indépendamment l'un de l'autre un atome d'hydrogène, un groupe alkyle en C₁-C₄, un groupe hydroxy(alkyle en C₁-C₄) ou un groupe aryl(alkyle en C₁-C₄) et q est égal à 1, 2, 3 ou 4, un groupe nitro, un groupe acide sulfonique, un groupe hydroxy, un groupe carboxyle, un groupe de formule II, dans laquelle A, R⁶, R⁷, R⁸ et R⁹ sont définis comme ci-dessus, ou un groupe de formule III,
deux radicaux contiguës de R², R³, R⁴ et R⁵ pouvant former un radical condensé, choisi parmi les formules IV à VIII, 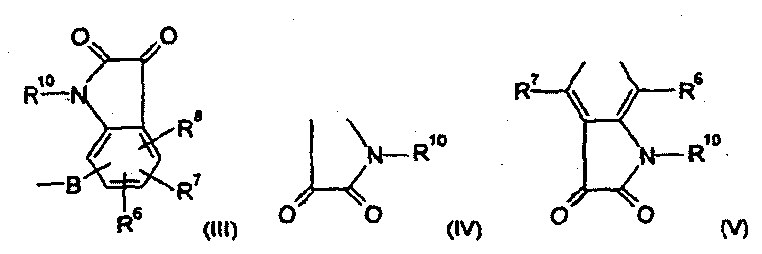 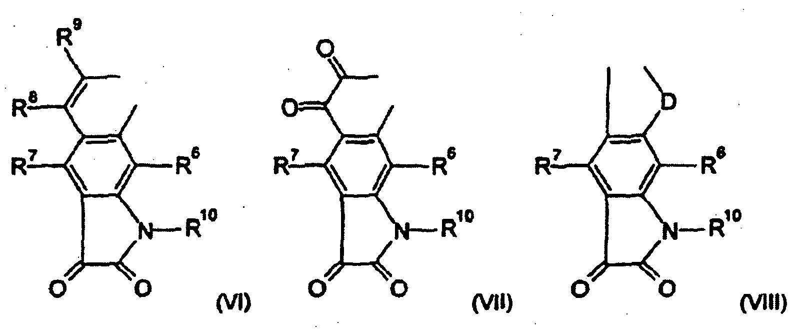 où
- R⁶, R⁷, R⁸ et R⁹ sont définis comme ci-dessus,
- R¹⁰ représente un atome d'hydrogène, un groupe alkyle en C₁-C₄, un groupe alcényle en C₂-C₄, un groupe aryle, un groupe aryl(alkyle en C₁-C₄), un groupe acyle en C₁-C₄, un groupe hydroxy(alkyle en C₁-C₄), un groupe dihydroxy(alkyle en C₂-C₄), un groupe sulfo(alkyle en C₁-C₄), un groupe carboxy(alkyle en C₁-C₄), un groupe R^{X}R^{XI}N-(CH₂)ₛ- ou R^{X}R^{XI}R^{XII}N⁺-(CH₂)ₛ-, où R^{X}, R^{XI} et R^{XII} représentent indépendamment l'un de l'autre un atome d'hydrogène, un groupe alkyle en C₁-C₄, un groupe hydroxy(alkyle en C₁-C₄) ou un groupe aryl(alkyle en C₁-C₄) et s est égal à 1, 2, 3 ou 4,
- B représente une liaison directe, un atome d'oxygène, un atome de soufre, un groupe disulfure, un groupe NH, un groupe sulfonyle, un groupe alkylène en C₁-C₈, un groupe carbonyle, un groupe -O-(CH₂)ₜ-O-, où t est égal à 1, 2, 3 ou 4, un groupe -(CH₂)ᵤ-O-(CH₂)_{V}-, où u et v représentent indépendamment l'un de l'autre un des nombres 1, 2, 3 ou 4,
- D représente un groupe méthylène, un groupe carbonyle et un groupe NH
à condition que soit l'un des radicaux R¹, R², R³, R⁴ ou R⁵ est un groupe de formule II ou III selon la définition indiquée ci-dessus, soit deux des radicaux contiguës parmi R², R³, R⁴ ou R⁵ forment conjointement un radical condensé, choisi par les formules IV à VIII.

2. Agent selon la revendication 1, **caractérisé en ce qu'**il contient en outre, en tant que composant B, au moins un composé, choisi parmi (a) des composés comprenant un groupe hydroxy ou un groupe amino primaire ou secondaire, choisis parmi les composés hydroxylés aromatiques, les amines aromatiques primaires ou secondaires et les composés hétérocycliques azotés, (b) les acides aminés, (c) les composés à CH acide.

3. Agent de teinture pour fibres kératiniques, en particulier de cheveux humains, contenant, en tant que teinture montant directement sur la fibre, au moins un produit de la réaction entre un composé de formule I selon la revendication 1 et un composé selon la revendication 2.

4. Agent selon l'une quelconque des revendications 1 à 3, **caractérisé en ce que** les composés de formule I sont choisis parmi la (1 H, 7H)-2,3,5,6-tétrahydropyrrolo[3,2-f]indolo-2,3,5,6-tétrone, la (1 H,6H)-2,3,7,8-tétrahydropyrrolo-[2',3':1,2]naphto[5,6-b]pyrrolo-2,3,7,8-tétrone, la (3H, 8H)-1,2,6,7-tétrahydro-5,10-dichloroindolo[7,6-g]indolo-1,2,6,7-tétrone, la 5,5'-oxy-bis(1 H-2,3-dihydroindolo-2,3-dione), la 6,6'-oxy-bis(1 H-2,3-dihydroindolo-2,3-dione), la 6,6'-carbonyl-bis-(1 H-2,3-dihydro-indolo-2,3-dione), la (1 H,7H)-2,3,5,6-tétrahydro-8-chloro-4-méthoxy-benzo[1,2-b:5,4-b']dipyrrolo-2,3,5,6-tétrone, la (1 H, 8H)-2,3,6,7-tétrahydro-benzo[2,1-b:3,4-b']dipyrrolo-2,3,6,7-tétrone, la 6,6'-méthylène-bis(1 H-2,3-tétrahydroindolo-2,3-dione), la 6,6'-méthylène-bis(1 H-2,3-tétrahydro-7-chloroindolo-2,3-dione), la 1,1'-méthytène-bis(1 H-2,3-tétrahydro-indolo-2,3-dione), la 1,1'-(1,3-propanediyl)-bis(1 H-2,3-tétrahydro-5-méthyl-indolo-2,3-dione), la (1H, 7H)-2,3,5,6-tétrahydro-8-méthylbenzo[1,2-b:5,4-b']dipyrrolo-2,3,5,6-tétrone, la (1H, 1'H)-2,2',3,3'-tétrahydro-7,7'-diméthoxy-5,5'-bündolo-2,2',3,3'-tétrone, la (1 H, 7H)-2,3,5,6-tétrahydro-4,8-diméthylbenzo[1,2-b:5,4-b'] dipyrrolo-2,3,5,6-tétrone, la (1 H, 1'H)-2,2',3,3'-tétrahydro-5,5',6,6'-tétrahydroxy-1,1'-diméthyl-4,4'-biindolo-2,2',3,3'-tétrone.

5. Agent selon l'une quelconque des revendications 1 à 4, **caractérisé en ce qu'**il contient les composés de formule I resp. les composés du composant B chacun dans une quantité de 0,03 à 65 mmoles, en particulier de 1 à 40 mmoles, par rapport à 100 g du poids total de l'agent de teinture.

6. Agent selon l'une quelconque des revendications 2 à 5, **caractérisé en ce que** les amines aromatiques primaires ou secondaires du composant B sont choisies dans le groupe formé par la N,N-diméthyl-p-phénylènediamine, la N,N-diéthyl-p-phénylènediamine, la N-(2-hydroxyéthyl)-N-éthyl-p-phénylène-diamine, la N,N-bis-(2-hydroxyéthyl)-p-phénylènediamine, la N-(2-méthoxyéthyl)-p-phénylènediamine, la 2,3-dichloro-p-phénylènediamine, la 2,4-dichloro-p-phénylènediamine, la 2,5-dichloro-p-phénylènediamine, la 2-chloro-p-phénylènediamine, la 2,5-dihydroxy-4-morpholinoaniline, le 2-aminophénol, le 3-aminophénol, le 4-aminophénol, le 2-aminométhyl-4-aminophénol, le 2-hydroxyméthyl-4-aminophénol, la o-phénylènediamine, la m-phénylènediamine, la p-phénylènediamine, le 2,5-diaminotoluène, le 2,5,-diaminophénol, le 2,5-diaminoanisol, le 2,5-diaminophénéthol, le 4-amino-3-méthylphénol, le 2-(2,5-diaminophényl)éthanol, le 2,4-diaminophénoxyéthanol, le 2-(2,5-diaminophénoxy)éthanol, le 3-amino-4-(2-hydroxyéthyloxy)phénol, le 3,4-méthylènedioxyphénol, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 3-amino-2-chloro-6-méthylphénol, le 2-méthyl-5-amino-4-chlorophénol, le 5-(2-hydroxy-éthylamino)-4-méthoxy-2-méthylphénol, le 4-amino-2-hydroxyméthylphénol, le 2-(diéthylaminométhyl)-4-aminophénol, le 4-amino-1-hydroxy-2-(2-hydroxyéthyl-aminométhyl)benzène, le 1-hydroxy-2-amino-5-méthylbenzène, le 1-hydroxy-2-amino-6-méthylbenzène, le 2-amino-5-acétamidophénol, le 1,3-diméthyl-2,5-diaminobenzène, le 5-(3-hydroxypropylamino-)-2-méthylphénol, le 5-amino-4-méthoxy-2-méthylphénol, le N,N-diméthyl-3-aminophénol, le N-cyclopentyl-3-aminophénol, le 5-amino-4-fluoro-2-méthylphénol, le 2,4-diamino-5-fluorotoluène, le 2,4-diamino-5-(2-hydroxyéthoxy)toluène, le 2,4-diamino-5-méthylphénétol, le 3,5-diamino-2-méthoxy-1-méthylbenzène, le 2-amino-4-(2-hydroxyéthylamino)anisol, le 2,6-bis-(2-hydroxyéthylamino)-1-méthylbenzène, le 1,3-diamino-2,4-diméthoxybenzène, le 3,5-diamino-2-méthoxytoluène, l'acide 2-aminobenzoïque, l'acide 3-aminobenzoïque, l'acide 4-aminobenzoïque, l'acide 2-aminophénylacétique, l'acide 3-aminophénylacétique, l'acide 4-aminophénylacétique, l'acide 2,3-diaminobenzoïque, l'acide 2,4-diaminobenzoïque, l'acide 2,5-diaminobenzoïque, l'acide 3,4-diaminobenzoïque, l'acide 3,5-diaminobenzoïque, l'acide 4-aminosalicylique, l'acide 5-aminosalicylique, l'acide 3-amino-4-hydroxybenzoïque, l'acide 4-amino-3-hydroxybenzoïque, l'acide 2-aminobenzènesulfonique, l'acide 3-aminobenzènesulfonique, l'acide 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophthalique, l'acide 5-aminoisophtalique, le 1,3,5-triaminobenzène, le 1,2,4-triaminobenzène, le 1,2,4,5-tétraaminobenzène, le 2,4,5-triaminophénol, le penta-aminobenzène, l'hexa-aminobenzène, le 2,4,6-triaminorésorcinol, le 4,5-diaminopyrocatéchinol, le 4,6-diaminopyrogallol, la 1-(2-hydroxy-5-aminobenzyl)-2-imidazolidinone, le 2,2'-(1,4-pipérazinylène-1,4-diméthylène)-bis-4-aminophénol, le 3,5-diamino-4-hydroxy-pyrocatéchinol, le 1,4-bis-(4-aminophényl)-1,4-diazacycloheptane, des nitriles aromatiques, tels que le 2-amino-4-hydroxybenzonitrile, le 4-amino-2-hydroxybenzonitrile, le 4-aminobenzonitrile, le 2,4-diaminobenzonitrile, des composés amino comprenant un groupe nitro, tels que la 3-amino-6-méthylamino-2-nitropyridine, l'acide picraminique, le chlorure de [8-[(4-amino-2-nitrophényl)-azo]-7-hydroxy-napht-2-yl]triméthylammonium, le chlorure de [8-((4-amino-3-nitrophényl)-azo)-7-hydroxy-napht-2-yl]triméthylammonium (Basic Brown 17), le 1-hydroxy-2-amino-4,6-dinitrobenzène, le 1-amino-2-nitro-4-[bis-(2-hydroxyéthyl)-amino]benzène, le 1-amino-2-[(2-hydroxyéthyl)amino]-5-nitrobenzène (HC Yellow Nr. 5), le 1-amino-2-nitro-4-[(2-hydroxyéthyl)amino]benzène (HC Red Nr. 7), la 2-chloro-5-nitro-N-2-hydroxyéthyl-1,4-phénylènediamine, le 1-[(2-hydroxyéthyl)amino]-2-nitro-4-aminobenzène (HC Red Nr. 3), le 4-amino-3-nitrophénol, le 4-amino-2-nitrophénol, la 6-nitro-o-toluidine, le 1-amino-3-méthyl-4-[(2-hydroxyéthyl)amino]-6-nitrobenzène (HC Violet Nr. 1), le 1-amino-2-nitro-4-[(2,3-dihydroxypropyl)amino]-5-chlorobenzène (HC Red Nr. 10), l'acide 2-(4-amino-2-nitroanilino)benzoïque, la 6-nitro-2,5-diaminopyridine, le 2-amino-6-chloro-4-nitrophénol, le sel disodique de l'acide 1-amino-2-(3-nitrophénylazo)-7-phénylazo-8-naphtol-3,6-disulfonique (Acid blue Nr. 29), le sel disodique de l'acide 1-amino-2-(2-hydroxy-4-nitrophénylazo)-8-naphtol-3,6-disulfonique (Palatinchrome green), le sel disodique de l'acide 1-amino-2-(3-chloro-2-hydroxy-5-nitrophénylazo)-8-naphtol-3,6-disulfonique (Gallion), le sel disodique de l'acide 4-amino-4'-nitrostilbène-2,2'-disulfonique, le 2,4-diamino-3',5'-dinitro-2'-hydroxy-5-méthylazobenzène (Mordant brown 4), l'acide 4'-amino-4-nitrodiphénylamin-2-sulfonique, l'acide 4'-amino-3'-nitrobenzophénon-2-carboxylique, le 1-amino-4-nitro-2-(2-nitrobenzylidèneamino)benzène, la 2-[2-(diéthylamino)éthylamino]-5-nitroaniline, l'acide 3-amino-4-hydroxy-5-nitrobenzènesulfonique, le 3-amino-3'-nitrobiphényle, le 3-amino-4-nitroacénaphtène, le 2-amino-1-nitronaphtalène, le 5-amino-6-nitrobenzo-1,3-dioxol, les anilines, en particulier les anilines comprenant un groupe nitro, telles que la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthylamino)benzène, le 2-nitro-1-amino-4-[bis-(2-hydroxyéthyl)-amino]benzène, l'acide 4-amino-2-nitrodiphénylamine-2'-carboxylique, le 1-amino-5-chloro-4-(2-hydroyéthylamino)-2-nitrobenzène, le 4,4'-diaminostilbène et son chlorhydrate, le sel mono- ou disodique de l'acide 4,4'-diaminostilbène-2,2'-disulfonique, le 4-amino-4'-diméthylaminostilbène et son chlorhydrate, le 4,4'-diaminodiphénylméthane, le 4,4'-diaminodiphénylsulfure, le 4,4'-diaminodiphénylsulfoxyde, la 4,4'-diaminodiphénylamine, l'acide 4,4'-diaminodiphénylamine-2-sulfonique, la 4,4'-diaminobenzophénone, le 4,4'-diaminodiphényléther, le 3,3',4,4'-tétraaminodiphényle, la 3,3',4,4'-tétraamino-benzophénone, le 1,3-bis-(2,4-diaminophénoxy)propane, le 1,8-bis-(2,5-diaminophénoxy)-3,6-dioxaoctane, le 1,3-bis-(4-aminophénylamino)propane, le 1,3-bis-(4-aminophénylamino)-2-propanol, le 1,3-bis-[N-(4-aminophényl)-2-hydroxyéthylamino]-2-propanol, la N,N-bis-[2-(4-aminophénoxy)-éthyl]méthylamine, la N-phényl-1,4-phénylènediamine et le bis-(5-amino-2-hydroxyphényl)méthane, ainsi que les sels physiologiquement acceptables des composés mentionnés ci-dessus.

7. Agent selon l'une quelconque des revendications 2 à 6, **caractérisé en ce que** les composés hétérocycliques azotés du composant B sont choisis dans le groupe formé par la 2-aminopyridine, la 3-aminopyridine, la 4-aminopyridine, la 2-amino-3-hydroxypyridine, la 2,6-diaminopyridine, la 2,5-diaminopyridine, la 2-(aminoéthylamino)-5-aminopyridine, la 2,3-diaminopyridine, la 2-diméthylamino-5-aminopyridine, la 2-méthylamino-3-amino-6-méthoxypyridine, la 2,3-diamino-6-méthoxypyridine, la 2,6-diméthoxy-3,5-diaminopyridine, la 2,4,5-triaminopyridine, la 2,6-dihydroxy-3,4-diméthylpyridine, la N-[2-(2,4-diaminophénylamino)-éthyl]-N-(5-amino-2-pyridyl)amine, la N-[2-(4-aminophényl)aminoéthyl]-N-(5-amino-2-pyridyl)amine, la 2,4-dihydroxy-5,6-diaminopyrimidine, la 4,5,6-triaminopyrimidine, la 4-hydroxy-2,5,6-triaminopyrimidine, la 2-hydroxy-4,5,6-triaminopyrimidine, la 2,4,5,6-tétra-aminopyrimidine, la 2-méthylamino-4,5,6-triaminopyrimidine, la 2,4-diaminopyrimidine, la 4,5-diaminopyrimidine, la 2-amino-4-méthoxy-6-méthylpyrimidine, le 3,5-diaminopyrazol, le 3,5-diamino-1,2,4-triazol, le 3-aminopyrazol, le 3-amino-5-hydroxypyrazol, le 1-phényl-4,5-diaminopyrazol, le 1-(2-hydroxyéthyl)-4,5-diaminopyrazol, le 1-phényl-3-méthyl-4,5-diaminopyrazol, la 4-amino-2,3-diméthyl-1-phényl-3-pyrazolin-5-one (4-aminoantipyrine), la 1-phényl-3-méthytpyrazol-5-one, la 2-aminoquinoléine, la 3-aminoquinoléine, la 8-aminoquinoléine, la 4-aminoquinaldine, l'acide 2-aminonicotinique, l'acide 6-aminonicotinique, la 5-aminoisoquinoléine, le 5-aminoindazole, le 6-aminoindazole, le 5-aminobenzimidazole, le 7-aminobenzimidazole, le 5-aminobenzothiazole, le 7-aminobenzothiazole, la 2,5-dihydroxy-4-morpholinoaniline ainsi que les dérivés d'indole et d'indoline, tels que le 4-aminoindole, le 5-aminoindole, le 6-aminoindole, le 7-aminoindole, le 5,6-dihydroxyindole, la 5,6-dihydroxyindoline, la 4-hydroxyindoline et les dérivés d'hydroxypyrimidine, ainsi que les sels physiologiquement acceptables des composés mentionnés ci-dessus.

8. Agent selon l'une quelconque des revendications 2 à 7, **caractérisé en ce que** les composés hydroxylés aromatiques du composant B sont choisis dans le groupe formé par le 2-méthylrésorcinol, le 4-méthylrésorcinol, le 5-méthylrésorcinol, le 2,5-diméthylrésorcinol, le résorcinol, le 3-méthoxyphénol, le pyrocatéchinol, l'hydroquinone, le pyrogallol, la phloroglucine, l'hydroxyhydroquinone, le 2-méthoxyphénol, le 3-méthoxyphénoi, le 4-méthoxyphénol, le 3-diméthylaminophénol, le 2-(2-hydroxyéthyl)phénol, le 3,4-méthylène-dioxyphénol, l'acide 2,4-dihydroxybenzoïque, l'acide 3,4-dihydroxybenzoïque, l'acide 2,4-dihydroxyphénylacétique, l'acide 3,4-dihydroxyphénylacétique, l'acide gallique, l'acide 2,4,6-trihydroxybenzoïque, la 2,4,6-trihydroxyacétophénone, le 2-chlororésorcinol, le 4-chlororésorcinol, le 1-naphtol, le 1,5-dihydroxynaphtalène, le 2,3-dihydroxynaphtalène, le 2,7-dihydroxynaphtalène, l'acide 6-diméthylamino-4-hydroxy-2-naphtalène- sulfonique et l'acide 3,6-dihydroxy-2,7-naphtalènesulfonique, ainsi que les sels physiologiquement acceptables des composés mentionnés ci-dessus.

9. Agent selon l'une quelconque des revendications 2 à 8, **caractérisé en ce que** les acides aminés du composant B sont choisis dans le groupe formé par l'arginine, l'histidine, la tyrosine, la phénylalanine, la DOPA (dihydroxyphénylalanine), l'ornithine, la proline, la lysine, le tryptophane, l'acide 6-aminocaproïque et la β-alanine ainsi que les sels physiologiquement acceptables des composés mentionnés ci-dessus.

10. Agent selon l'une quelconque des revendications 2 à 9, **caractérisé en ce que** les composés à CH acide du composant B sont choisis dans le groupe formé par l'iodure de 1,2,3,3-tétraméthyl-3H-indolium, le p-toluènesulfonate de 1,2,3,3-tétraméthyl-3H-indolium, le méthansulfonate de 1,2,3,3-tétraméthyl-3H-indolium, la 1,3,3-triméthyl-2-méthylèneindoline (base de Fischer), l'iodure de 2,3-diméthylbenzothiazolium, le p-toluènesulfonate de 2,3-diméthyl-benzothiazolium, le p-toluènesulfonate de 1,2-diméthyl-naphto[1,2-d]thiazolium, le p-toluènesulfonate de 1-éthyl-2-méthyl-naphto[1,2-d]thiazolium, la rhodanine, l'acide rhodanine-3-acétique, l'iodure de 1-méthyl-2-quinaldinium, le p-toluènesulfonate de 1-méthyl-2-quinaldinium, l'iodure de 1-éthyl-2-quinaldinium, le p-toluènesulfonate de 1-éthyl-2-quinaldinium, l'iodure de 1,4-diméthylquinolinium, le p-toluènesulfonate de 1,4-diméthylquinolinium, l'acide barbiturique, l'acide thiobarbiturique, l'acide 1,3-diméthylthiobarbiturique, l'acide 1,3-diéthylthiobarbiturique, l'oxindote, l'acétate de 3-indoxyle, la 2-coumaranone, la 5-hydroxycoumaranone, la 6-hydroxycoumaranone, la 1-méthyl-3-phényl-2-pyrazolinone, l'indane-1,3-dione, l'indane-1-one, le benzoylacétonitrile, le 1-dicyanométhylèneindane, le 1,3-bis(dicyanométhylène)indane, la 3-dicyanométhylèneindan-1-one, la 1,3-diiminoisoindoline, le chlorhydrate de 2-amino-4-imino-1,3-thiazoline, la 3-cyano-1,4-diméthyl-6-hydroxy-2-pyridone et la 3-cyano-1-éthyl-6-hydroxy-4-méthyl-2-pyridone, ainsi que les sels physiologiquement acceptables des composés mentionnés ci-dessus.

11. Agent selon l'une quelconque des revendications 2 à 10, **caractérisé en ce que** le composant B est choisi parmi les composés du groupe constitué par la N-(2-hydroxyéthyl)-N-éthyl-p-phénylènediamine, la 2-chloro-p-phénylènediamine, la N,N-bis-(2-hydroxyéthyl)-p-phénylènediamine, le 2-aminophénol, le 3-aminophénol, le 4-aminophénol, le 2-amino-6-chloro-4-nitrophénol, la p-phénylènediamine, le 2-(2,5-diaminophényl)éthanol, le 2,5-diaminotoluène, la 3,4-méthylènedioxyaniline, le 2-amino-4-(2-hydroxy-éthylamino)anisol, le 2-(2,4-diaminophénoxy)éthanol, le 3-amino-2,4-dichlorophénol, le 2-méthyl-5-aminophénol, le 3-méthyl-4-aminophénol, le 2-méthyl-5-(2-hydroxyéthylamino)phénol, le 2-méthyl-5-amino-4-chlorophénol, le 6-méthyl-3-amino-2-chlorophénol, le 2-aminométhyl-4-aminophénol, le 2-diéthylaminométhyl-4-aminophénol, le 2-diméthylaminométhyl-4-aminophénol, le 2,6-dichloro-4-aminophénol, le 2-hydroxyméthyl-4-aminophénol, le 2,6-bis(2-hydroxyéthylamino)-1-méthylbenzène, le bis-(2-hydroxy-5-aminophényl)méthane, le bis-(4,5-amino-2-hydroxyphényl)méthane, le 1,3-bis(2,4-diamino-phénoxy)propane, le 1,4-bis(4-aminophényl)-1,4-diazacycloheptane, le 1,8-bis(2,5-diaminophénoxy)-3,6-dioxaoctane, la 4,4'-diaminodiphénylamine, le 3,4-méthylènedioxyphénol, l'acide 3,4-diaminobenzoïque, la 2,5-diaminopyridine, la 2-diméthylamino-5-aminopyridine, la 2-amino-3-hydroxypyridine, la 3-amino-2-méthylamino-6-méthoxypyridine, la 2,3-diamino-6-méthoxypyridine, la 3,5-diamino-2,6-diméthoxypyridine, la 2,6-dihydroxy-3,4-diméthylpyridine, la 2-hydroxy-4,5,6-triaminopyrimidine, la 4-hydroxy-2,5,6-triaminopyrimidine, la 2,4,5,6-tétra-aminopyrimidine, la 2-méthylamino-4,5,6-triaminopyrimidine, le 3,5-diaminopyrazole, le 3-amino-5-hydroxypyrazole, le 4,5-diamino-1-(2-hydroxyéthyl)pyrazole, le 5,6-dihydroxyindole, la 5,6-dihydroxyindoline, la 4-amino-2,3-diméthyl-1-phényl-3-pyrazolin-5-one (4-aminoantipyrine), le p-toluènesulfonate de 2,3-diméthylbenzothiazolium, le p-toluènesulfonate de 1,2,3,3-tétraméthyl-3H-indolinium, l'acide thiobarbiturique, la rhodanine, le p-toluènesulfonate de 2,3-diméthyl-benzothiazolium, le p-toluènesulfonate de 1,2-diméthyl-naphto[1,2-d]thiazolium, le p-toluènesulfonate de 1-méthyl-2-quinaldinium, le p-toluènesulfonate de 1,4-diméthylquinolinium, la β-alanine, la L-proline, la L-lysine, la DL-tyrosine, ainsi que les sels physiologiquement acceptables, formés de préférence avec des acides inorganiques, des composés mentionnés ci-dessus.

12. Agent selon l'une quelconque des revendications 1 à 11, **caractérisé en ce qu'**il contient un renforçateur pour la teinture choisi dans le groupe formé par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'histidine, la pyrrolidine, la proline, la pyrrolidone, l'acide pyrrolidone-5-carboxylique, le pyrazole, le 1,2,4-triazole, la pipérazidine ou les mélanges quelconques de ces composés.

13. Agent selon l'une quelconque des revendications 1 à 12, **caractérisé en ce qu'**il contient des teintures montant directement sur la fibre des groupes formés des nitrophénylènediamines, des nitroaminophénols, des anthraquinones ou des indophénols, de préférence dans une quantité de 0,01 à 20 % en poids par rapport au poids total de la teinture

14. Agent selon l'une quelconque des revendications 1 à 13, **caractérisé en ce qu'**on y ajoute des sels d'ammonium ou des sels métalliques choisis dans le groupe formé par les formiates, les carbonates, les halogénures, les sulfates, les butyrates, les valérates, les caproates, les acétates, les lactates, les glycolates, les tartrates, les citrates, les gluconates, les propionates, les phosphates et les phosphonates de métaux alcalins, tels que le potassium, le sodium ou le lithium, de métaux alcalino- terreux, tels que le magnésium, le calcium, le strontium ou le baryum, ou d'aluminium, de manganèse, de fer, de cobalt, de cuivre ou de zinc.

15. Agent selon l'une quelconque des revendications 1 à 14, **caractérisé en ce qu'**il contient des agents d'oxydation, en particulier H₂O₂, dans une quantité de 0,01 à 6 % en poids par rapport à la solution d'application.

16. Agent selon l'une quelconque des revendications 1 à 15, **caractérisé en ce qu'**il contient des tensioactifs anioniques, zwitterioniques ou non ioniques.

17. Utilisation de
(a) au moins un composé selon la revendication 1, éventuellement en combinaison avec au moins un composant B selon la revendication 2,
et/ou
(b) au moins un produit de la réaction entre le composé selon la revendication 1 et le composant B
en tant que composant colorant dans une teinture capillaire.

18. Procédé pour la teinture de fibres kératiniques, en particulier de cheveux humains, dans lequel on applique sur les fibres kératiniques une teinture qui contient un composé selon la revendication 1, ainsi que des ingrédients cosmétiques usuels, on le laisse sur les fibres un certain temps, généralement environ 30 minutes, et ensuite, on l'élimine par rinçage ou lavage à l'aide d'un shampooing.

19. Procédé selon la revendication 18, **caractérisé en ce que** l'on applique en outre sur les fibres kératiniques un composant B qui contient au moins un composé choisi parmi (a) les composés contenant des groupes hydroxy ou des groupes amino primaires ou secondaires, choisis parmi les composés aromatiques hydroxylés, les amines aromatiques primaires ou secondaires et les composés hétérocycliques azotés, (b) les acides aminés, (c) les composés à CH acide, ainsi que des ingrédients cosmétiques usuels, on le laisse sur les fibres un certain temps, généralement environ 30 minutes, et ensuite, on l'élimine par rinçage ou lavage à l'aide d'un shampooing.
